(19) Europäisches Patentamt
European Patent Office
Office européen des brevets

(11) EP 3 845 554 A1

(12) EUROPEAN PATENT APPLICATION

(43) Date of publication:
**07.07.2021 Bulletin 2021/27**

(51) Int Cl.:
***C07K 14/47*** *(2006.01)* ***G01N 33/53*** *(2006.01)*

(21) Application number: **19220082.2**

(22) Date of filing: **30.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Technische Universität Dortmund**
**44227 Dortmund (DE)**

(72) Inventors:
- **SUMMERER, Daniel**
  **44137 Dortmund (DE)**
- **BUCHMULLER, Benjamin**
  **44379 Dortmund (DE)**

(74) Representative: **Viering, Jentschura & Partner mbB**
**Patent- und Rechtsanwälte**
**Hamborner Straße 53**
**40472 Düsseldorf (DE)**

# (54) METHOD FOR DETERMINING 5-METHYLCYTOSINE CONFIGURATIONS IN DNA

(57) The present invention relates to an isolated Methyl-CpG binding domain (MBD) variant comprising an MBD core domain that has at least 60 % sequence homology relative to any one of SEQ ID Nos. 1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40 in SEQ ID NO:1. Furthermore, the invention relates to a conjugate comprising the isolated MBD variant and to the use of the isolated MBD variant or the conjugate for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC). Finally, the invention relates to methods for the determination of the methylation state of cytosine and/or the relative oxidation state of the methyl group of 5-methylated cytosine residues as described above or for the enrichment of DNA molecules as described above.

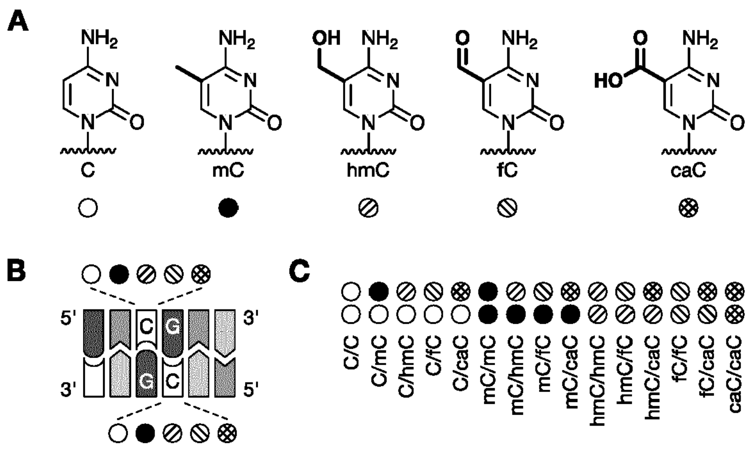


FIG. 1

EP 3 845 554 A1

SEQUENCE LISTING

```
<110>  Technische Universitaet Dortmund

<120>  METHOD FOR DETERMINING 5-METHYLCYTOSINE CONFIGURATIONS
       IN DNA

<130>  P75419

<160>  61

<170>  PatentIn version 3.5

<210>  1
<211>  45
<212>  PRT
<213>  Homo sapiens

<400>  1

Pro Ala Leu Gly Pro Gly Trp Lys Arg Arg Glu Val Phe Arg Lys Ser
1               5                   10                  15


Gly Ala Thr Cys Gly Arg Ser Asp Thr Tyr Tyr Gln Ser Pro Thr Gly
            20                  25                  30


Asp Arg Ile Arg Ser Lys Val Glu Leu Thr Arg Tyr Leu
        35                  40                  45


<210>  2
<211>  45
<212>  PRT
<213>  Homo sapiens

<400>  2

Pro Ala Leu Pro Pro Gly Trp Lys Lys Glu Glu Val Ile Arg Lys Ser
1               5                   10                  15


Gly Leu Ser Ala Gly Lys Ser Asp Val Tyr Tyr Phe Ser Pro Ser Gly
            20                  25                  30


Lys Lys Phe Arg Ser Lys Pro Gln Leu Ala Arg Tyr Leu
        35                  40                  45


<210>  3
<211>  45
<212>  PRT
<213>  Homo sapiens

<400>  3

Pro Ala Leu Pro Gln Gly Trp Glu Arg Glu Glu Val Pro Arg Arg Ser
1               5                   10                  15


Gly Leu Ser Ala Gly His Arg Asp Val Phe Tyr Tyr Ser Pro Ser Gly
```

20 25 30

Lys Lys Phe Arg Ser Lys Pro Gln Leu Ala Arg Tyr Leu
35 40 45

<210> 4
<211> 45
<212> PRT
<213> Homo sapiens

<400> 4

Lys Ser Val Pro Cys Gly Trp Glu Arg Val Val Lys Gln Arg Leu Phe
1 5 10 15

Gly Lys Thr Ala Gly Arg Phe Asp Val Tyr Phe Ile Ser Pro Gln Gly
20 25 30

Leu Lys Phe Arg Ser Lys Ser Ser Leu Ala Asn Tyr Leu
35 40 45

<210> 5
<211> 45
<212> PRT
<213> Homo sapiens

<400> 5

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile Asn Pro Gln Gly
20 25 30

Lys Ala Phe Arg Ser Lys Val Glu Leu Ile Ala Tyr Phe
35 40 45

<210> 6
<211> 45
<212> PRT
<213> Mus musculus

<400> 6

Pro Ala Leu Gly Pro Gly Trp Lys Arg Arg Glu Ser Phe Arg Lys Ser
1 5 10 15

Gly Ala Ser Phe Gly Arg Ser Asp Ile Tyr Tyr Gln Ser Pro Thr Gly
20 25 30

Glu Lys Ile Arg Ser Lys Val Glu Leu Thr Arg Tyr Leu
35 40 45

<210> 7
<211> 45
<212> PRT
<213> Mus musculus

<400> 7

```
Pro Ala Leu Pro Pro Gly Trp Lys Lys Glu Glu Val Ile Arg Lys Ser
1               5                   10                  15

Gly Leu Ser Ala Gly Lys Ser Asp Val Tyr Tyr Phe Ser Pro Ser Gly
            20                  25                  30

Lys Lys Phe Arg Ser Lys Pro Gln Leu Ala Arg Tyr Leu
        35                  40                  45
```

<210> 8
<211> 45
<212> PRT
<213> Mus musculus

<400> 8

```
Pro Ala Leu Pro Gln Gly Trp Glu Arg Glu Glu Val Pro Arg Arg Ser
1               5                   10                  15

Gly Leu Ser Ala Gly His Arg Asp Val Phe Tyr Tyr Ser Pro Ser Gly
            20                  25                  30

Lys Lys Phe Arg Ser Lys Pro Gln Leu Ala Arg Tyr Leu
        35                  40                  45
```

<210> 9
<211> 45
<212> PRT
<213> Mus musculus

<400> 9

```
Lys Pro Val Pro Cys Gly Trp Glu Arg Val Val Lys Gln Arg Leu Ser
1               5                   10                  15

Gly Lys Thr Ala Gly Lys Phe Asp Val Tyr Phe Ile Ser Pro Gln Gly
            20                  25                  30

Leu Lys Phe Arg Ser Lys Arg Ser Leu Ala Asn Tyr Leu
        35                  40                  45
```

<210> 10
<211> 45
<212> PRT
<213> Mus musculus

<400> 10

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile Asn Pro Gln Gly
20 25 30

Lys Ala Phe Arg Ser Lys Val Glu Leu Ile Ala Tyr Phe
35 40 45

<210> 11
<211> 45
<212> PRT
<213> Rattus norvegicus

<400> 11

Pro Ala Leu Gly Pro Gly Trp Lys Arg Arg Glu Ala Phe Arg Lys Ser
1 5 10 15

Gly Ala Ser Cys Gly Arg Ser Asp Ile Tyr Tyr Arg Ser Pro Thr Gly
20 25 30

Glu Lys Ile Arg Ser Lys Ile Glu Leu Thr Arg Tyr Leu
35 40 45

<210> 12
<211> 45
<212> PRT
<213> Rattus norvegicus

<400> 12

Pro Ala Leu Pro Pro Gly Trp Lys Lys Glu Glu Val Ile Arg Lys Ser
1 5 10 15

Gly Leu Ser Ala Gly Lys Ser Asp Val Tyr Tyr Phe Ser Pro Ser Gly
20 25 30

Lys Lys Phe Arg Ser Lys Pro Gln Leu Ala Arg Tyr Leu
35 40 45

<210> 13
<211> 45
<212> PRT
<213> Rattus norvegicus

<400> 13

Pro Ala Leu Pro Gln Gly Trp Glu Arg Glu Glu Val Pro Arg Arg Ser
1 5 10 15

Gly Leu Ser Ala Gly His Arg Asp Val Phe Tyr Tyr Ser Pro Ser Gly
20 25 30

Lys Lys Phe Arg Ser Lys Pro Gln Leu Ala Arg Tyr Leu
35 40 45

<210> 14
<211> 45
<212> PRT
<213> Pan troglodytes

<400> 14

Lys Ser Val Pro Cys Gly Trp Glu Arg Val Val Lys Gln Arg Leu Phe
1 5 10 15

Gly Lys Thr Ala Gly Arg Phe Asp Val Tyr Phe Ile Ser Pro Gln Gly
20 25 30

Leu Lys Phe Arg Ser Lys Ser Ser Leu Ala Asn Tyr Leu
35 40 45

<210> 15
<211> 45
<212> PRT
<213> Pan troglodytes

<400> 15

Pro Ala Leu Gly Pro Gly Trp Lys Arg Arg Glu Val Phe Arg Lys Ser
1 5 10 15

Gly Ala Thr Cys Gly Arg Ser Asp Thr Tyr Tyr Gln Ser Pro Thr Gly
20 25 30

Asp Arg Ile Arg Ser Lys Val Glu Leu Thr Arg Tyr Leu
35 40 45

<210> 16
<211> 45
<212> PRT
<213> Pan troglodytes

<400> 16

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile Asn Pro Gln Gly
20 25 30

Lys Ala Phe Arg Ser Lys Val Glu Leu Ile Ala Tyr Phe
35 40 45

<210> 17
<211> 45
<212> PRT
<213> Pan troglodytes

<400> 17

Pro Thr Leu Pro Glu Gly Trp Thr Arg Lys Leu Lys Gln Arg Lys Ser
1               5                   10                  15

Gly Arg Ser Ala Gly Lys Tyr Asp Val Tyr Leu Ile Asn Pro Gln Gly
            20                  25                  30

Lys Ala Phe Arg Ser Lys Val Glu Leu Ile Ala Tyr Phe
        35                  40                  45

<210> 18
<211> 45
<212> PRT
<213> Danio rerio

<400> 18

Pro Ser Leu Pro Gln Gly Trp Thr Arg Lys Leu Lys Gln Arg Lys Ser
1               5                   10                  15

Gly Arg Ser Ala Gly Lys Phe Asp Val Tyr Leu Ile Asn Pro Glu Gly
            20                  25                  30

Lys Ala Phe Arg Ser Lys Val Glu Leu Met Ala Tyr Phe
        35                  40                  45

<210> 19
<211> 45
<212> PRT
<213> Danio rerio

<400> 19

Ser Ala Leu Pro Asn Gly Trp Lys Met Glu Glu Val Thr Arg Lys Ser
1               5                   10                  15

Gly Leu Ser Ala Gly Lys Ser Asp Val Tyr Tyr Phe Ser Pro Thr Gly
            20                  25                  30

Lys Lys Phe Arg Ser Lys Pro Gln Leu Val Arg Tyr Leu
        35                  40                  45

<210> 20
<211> 45
<212> PRT
<213> Takifugu rubripes

<400> 20

Thr Ala Leu Pro Lys Gly Trp Lys Met Glu Glu Val Thr Arg Lys Ser
1 5 10 15

Gly Leu Ser Ala Gly Lys Ser Asp Val Tyr Tyr Phe Ser Pro Ser Gly
20 25 30

Lys Lys Phe Arg Ser Lys Pro Gln Leu Ala Arg Tyr Leu
35 40 45

<210> 21
<211> 45
<212> PRT
<213> Xenopus laevis

<400> 21

Pro Thr Leu Pro Glu Gly Trp Thr Arg Lys Leu Lys Gln Arg Lys Ser
1 5 10 15

Gly Arg Ser Ala Gly Lys Phe Asp Val Tyr Leu Ile Asn Pro Asn Gly
20 25 30

Lys Ala Phe Arg Ser Lys Val Glu Leu Ile Ala Tyr Phe
35 40 45

<210> 22
<211> 45
<212> PRT
<213> Xenopus laevis

<400> 22

Ser Ala Leu Pro Gln Gly Trp Lys Lys Glu Glu Val Thr Arg Arg Ser
1 5 10 15

Gly Leu Ser Ala Gly Lys Ser Asp Val Tyr Tyr Tyr Ser Pro Ser Gly
20 25 30

Lys Lys Phe Arg Ser Lys Pro Gln Leu Ala Arg Tyr Leu
35 40 45

<210> 23
<211> 45
<212> PRT
<213> Xenopus laevis

<400> 23

Pro Ala Leu Pro Ala Gly Trp Lys Lys Glu Glu Val Ile Arg Lys Ser
1 5 10 15

Gly Leu Ser Ala Gly Lys Ser Asp Val Tyr Tyr Tyr Ser Pro Asn Gly
20 25 30

Lys Lys Phe Arg Ser Lys Pro Gln Leu Ala Arg Tyr Leu
35 40 45

<210> 24
<211> 45
<212> PRT
<213> Xenopus laevis

<400> 24

Pro Leu Leu Gly Pro Gly Trp Lys Arg Arg Asn Val Val Arg Lys Ser
1 5 10 15

Gly Ala Thr Cys Gly His Ser Asp Thr Tyr Tyr Arg Ser Pro Ala Gly
20 25 30

Lys Lys Ile Arg Ser Arg Ile Glu Leu Ala Lys Tyr Leu
35 40 45

<210> 25
<211> 45
<212> PRT
<213> Xenopus laevis

<400> 25

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile His Pro Asn Gly
20 25 30

Lys Ala Phe Arg Ser Lys Val Glu Leu Ile Ala Tyr Phe
35 40 45

<210> 26
<211> 45
<212> PRT
<213> Gallus gallus

<400> 26

Pro Ala Leu Pro Pro Gly Trp Lys Lys Glu Glu Val Ile Arg Lys Ser
1 5 10 15

Gly Leu Ser Ala Gly Lys Ser Asp Val Tyr Tyr Phe Ser Pro Ser Gly
20 25 30

Lys Lys Phe Arg Ser Lys Pro Gln Leu Ala Arg Tyr Leu

35 40 45

<210> 27
<211> 45
<212> PRT
<213> Strongylocentrotus purpuratus

<400> 27

Pro Gly Leu Pro Ala Gly Trp Lys Arg Glu Glu Val Ile Arg Lys Ser
1 5 10 15

Gly Leu Ser Ala Gly Lys Thr Asp Val Tyr Tyr Tyr Ser Pro Cys Gly
20 25 30

Lys Lys Leu Arg Ser Lys Pro Gln Leu Ala Arg Phe Ile
35 40 45

<210> 28
<211> 46
<212> PRT
<213> Strongylocentrotus purpuratus

<400> 28

Trp Pro Leu Ala Asn Gly Trp Arg Arg Gln Thr Ile Ile Arg Gln Leu
1 5 10 15

Gly Pro Gly Asp Arg Ile Lys Gly Asp Val Ile Tyr Tyr Ala Pro Cys
20 25 30

Gly Lys Lys Leu Arg Thr Tyr Pro Glu Val Val Arg Tyr Ile
35 40 45

<210> 29
<211> 45
<212> PRT
<213> Anopheles gambiae

<400> 29

Ala Ala Leu Pro Lys Gly Trp Gln Arg Glu Glu Val Leu Arg Lys Thr
1 5 10 15

Gly Leu Ser Ala Gly Lys Val Asp Val Tyr Tyr Tyr Ser Pro Thr Gly
20 25 30

Lys Lys Ile Glu Ser Lys Pro Gln Leu Ala Arg Ala Leu
35 40 45

<210> 30
<211> 45
<212> PRT

<213> Caenorhabditis elegans

<400> 30

Leu Pro Leu Gln Leu Gly Trp Arg Arg Gln Thr Cys Val Arg Ser Ile
1 5 10 15

Ala Ser Ala Gly Val Lys Gly Asp Val Ser Tyr Phe Ala Pro Cys Gly
20 25 30

Lys Lys Leu Ser Thr Tyr Ser Glu Val Val Arg Tyr Leu
35 40 45

<210> 31
<211> 42
<212> PRT
<213> Arabidopsis thaliana

<400> 31

Pro Lys Thr Pro Lys Gly Phe Lys Arg Ser Leu Val Leu Arg Lys Asp
1 5 10 15

Tyr Ser Lys Met Asp Thr Tyr Tyr Phe Thr Pro Thr Gly Lys Lys Leu
20 25 30

Arg Ser Arg Asn Glu Ile Ala Ala Phe Val
35 40

<210> 32
<211> 42
<212> PRT
<213> Arabidopsis thaliana

<400> 32

Pro Arg Thr Pro Arg Gly Phe Lys Arg Ser Leu Ile Leu Arg Lys Asp
1 5 10 15

Tyr Ser Lys Met Asp Ala Tyr Tyr Ile Thr Pro Thr Gly Lys Lys Leu
20 25 30

Lys Ser Arg Asn Glu Ile Ala Ala Phe Ile
35 40

<210> 33
<211> 44
<212> PRT
<213> Homo sapiens

<400> 33

Leu Pro Leu Gln His Gly Trp Arg Arg Glu Val Arg Ile Lys Lys Gly
1 5 10 15

Ser His Arg Trp Gln Gly Glu Thr Trp Tyr Tyr Gly Pro Cys Gly Lys
20 25 30

Arg Met Lys Gln Phe Pro Glu Val Ile Lys Tyr Leu
35 40

<210> 34
<211> 44
<212> PRT
<213> Homo sapiens

<400> 34

Ile Pro Leu Glu Tyr Gly Trp Gln Arg Glu Thr Arg Ile Arg Asn Phe
1 5 10 15

Gly Gly Arg Leu Gln Gly Glu Val Ala Tyr Tyr Ala Pro Cys Gly Lys
20 25 30

Lys Leu Arg Gln Tyr Pro Glu Val Ile Lys Tyr Leu
35 40

<210> 35
<211> 44
<212> PRT
<213> Homo sapiens

<400> 35

Leu Pro Leu Gln His Gly Trp Arg Arg Glu Val Arg Ile Lys Lys Gly
1 5 10 15

Ser His Arg Trp Gln Gly Glu Thr Trp Tyr Tyr Gly Pro Cys Gly Lys
20 25 30

Arg Met Lys Gln Phe Pro Glu Val Ile Lys Tyr Leu
35 40

<210> 36
<211> 44
<212> PRT
<213> Gallus gallus

<400> 36

Phe Pro Leu Gln His Gly Trp Arg Arg Glu Val Arg Ile Lys Arg Gly
1 5 10 15

Asn His Arg Trp Gln Gly Glu Thr Trp Tyr Tyr Gly Pro Cys Gly Lys
20 25 30

Arg Met Lys Gln Phe Pro Glu Val Ile Lys Tyr Leu
35 40

<210> 37
<211> 44
<212> PRT
<213> Rattus norvegicus

<400> 37

Leu Pro Leu Gln His Gly Trp Arg Arg Glu Val Arg Ile Lys Lys Gly
1 5 10 15

Ser His Arg Trp Gln Gly Glu Thr Trp Tyr Tyr Gly Pro Cys Gly Lys
20 25 30

Arg Met Lys Gln Phe Pro Glu Val Ile Lys Tyr Leu
35 40

<210> 38
<211> 44
<212> PRT
<213> Homo sapiens

<400> 38

Val Pro Leu Leu Tyr Asp Phe Arg Arg Met Thr Ala Arg Arg Arg Val
1 5 10 15

Asn Arg Lys Met Gly Phe His Val Ile Tyr Lys Thr Pro Cys Gly Leu
20 25 30

Cys Leu Arg Thr Met Gln Glu Ile Glu Arg Tyr Leu
35 40

<210> 39
<211> 43
<212> PRT
<213> Homo sapiens

<400> 39

Leu Pro Ile Lys Cys His Phe Gln Arg Arg His Ala Lys Thr Asn Ser
1 5 10 15

His Ser Ser Ala Leu His Val Ser Tyr Lys Thr Pro Cys Gly Arg Ser
20 25 30

Leu Arg Asn Val Glu Glu Val Phe Arg Tyr Leu
35 40

<210> 40
<211> 44

<212> PRT
<213> Homo sapiens

<400> 40

Val Pro Leu Leu Tyr Asp Phe Arg Arg Met Thr Ala Arg Arg Arg Val
1 5 10 15

Asn Arg Lys Met Gly Phe His Val Ile Tyr Lys Thr Pro Cys Gly Leu
20 25 30

Cys Leu Arg Thr Met Gln Glu Ile Glu Arg Tyr Leu
35 40

<210> 41
<211> 43
<212> PRT
<213> Homo sapiens

<400> 41

Leu Pro Ile Arg Cys His Phe Gln Arg Arg His Ala Lys Thr Asn Ser
1 5 10 15

His Ser Ser Ala Leu His Val Asn Tyr Lys Thr Pro Cys Gly Arg Asn
20 25 30

Leu Arg Asn Met Glu Glu Val Phe His Tyr Leu
35 40

<210> 42
<211> 44
<212> PRT
<213> Takifugu rubripes

<400> 42

Thr Pro Leu Leu Tyr Asp Phe Arg Arg Met Thr Gly Arg Arg Lys Val
1 5 10 15

Asn Arg Lys Met Ser Phe His Val Ile Tyr Lys Ala Pro Cys Gly Leu
20 25 30

Cys Leu Arg Asn Met Ser Glu Ile Gln His Tyr Leu
35 40

<210> 43
<211> 44
<212> PRT
<213> Xenopus laevis

<400> 43

Val Pro Leu Leu Tyr Asp Phe Arg Arg Met Thr Ala Arg Arg Arg Val

```
1               5                   10                  15

Asn Arg Lys Met Gly Phe His Val Ile Tyr Lys Ser Pro Cys Gly Leu
            20                  25                  30

Ser Leu Arg Thr Met Pro Glu Ile Glu Arg Tyr Leu
        35                  40


<210>  44
<211>  44
<212>  PRT
<213>  Gallus gallus

<400>  44

Ile Pro Leu Leu Tyr Asp Phe Arg Arg Met Thr Ala Arg Arg Arg Val
1               5                   10                  15

Asn Arg Lys Met Gly Phe His Val Ile Tyr Lys Thr Pro Cys Gly Leu
            20                  25                  30

Cys Leu Arg Ser Met Ala Glu Ile Glu Arg Tyr Leu
        35                  40


<210>  45
<211>  44
<212>  PRT
<213>  Rattus norvegicus

<400>  45

Val Pro Leu Leu Tyr Asp Phe Arg Arg Met Thr Ala Arg Arg Arg Val
1               5                   10                  15

Asn Arg Lys Met Gly Phe His Val Ile Tyr Lys Thr Pro Cys Gly Leu
            20                  25                  30

Cys Leu Arg Thr Met Gln Glu Ile Glu Arg Tyr Leu
        35                  40


<210>  46
<211>  45
<212>  PRT
<213>  Artificial

<220>
<223>  MBD variant of SEQ ID NO:5 (F105)

<400>  46

Pro Thr Leu Pro Glu Gly Trp Thr Arg Lys Leu Thr Gln Arg Lys Ser
1               5                   10                  15
```

```
Gly Arg Ser Ala Gly Lys Tyr Asp Thr Thr Leu Ile Asn Pro Gln Gly
            20                  25                  30

Lys Ala Phe Arg Lys Lys Val Glu Leu Ile Ala Tyr Phe
        35                  40                  45


<210>  47
<211>  45
<212>  PRT
<213>  Artificial

<220>
<223>  MBD variant of SEQ ID NO:5 (F106)

<400>  47

Pro Thr Leu Pro Glu Gly Trp Thr Arg Lys Leu Thr Gln Arg Lys Ser
1               5                   10                  15

Gly Arg Ser Ala Gly Lys Tyr Asp Ala Tyr Leu Ile Asn Pro Gln Gly
            20                  25                  30

Lys Ala Phe Arg Asn Lys Val Glu Leu Ile Ala Tyr Phe
        35                  40                  45


<210>  48
<211>  45
<212>  PRT
<213>  Artificial

<220>
<223>  MBD variant of SEQ ID NO:5 (F271)

<400>  48

Pro Thr Leu Pro Glu Gly Trp Thr Arg Lys Leu Lys Gln Arg Lys Ser
1               5                   10                  15

Gly Arg Ser Ala Gly Lys Tyr Asp Cys Ser Leu Ile Asn Pro Gln Gly
            20                  25                  30

Lys Ala Phe Arg Gln Lys Val Glu Leu Ile Ala Tyr Phe
        35                  40                  45


<210>  49
<211>  45
<212>  PRT
<213>  Artificial

<220>
<223>  MBD variant of SEQ ID NO:5 (F272)

<400>  49

Pro Thr Leu Pro Glu Gly Trp Thr Arg Lys Leu Ala Gln Arg Lys Ser
```

```
1               5                   10                  15

Gly Arg Ser Ala Gly Lys Tyr Asp Cys Phe Leu Ile Asn Pro Gln Gly
            20                  25                  30

Lys Ala Phe Arg Arg Lys Val Glu Leu Ile Ala Tyr Phe
        35                  40                  45
```

<210> 50
<211> 45
<212> PRT
<213> Artificial

<220>
<223> MBD variant of SEQ ID NO:5 (F273)

<400> 50

```
Pro Thr Leu Pro Glu Gly Trp Thr Arg Lys Leu Lys Gln Arg Lys Ser
1               5                   10                  15

Gly Arg Ser Ala Gly Lys Tyr Asp Leu Thr Leu Ile Asn Pro Gln Gly
            20                  25                  30

Lys Ala Phe Arg Arg Lys Val Glu Leu Ile Ala Tyr Phe
        35                  40                  45
```

<210> 51
<211> 45
<212> PRT
<213> Artificial

<220>
<223> MBD variant of SEQ ID NO:5 (F274)

<400> 51

```
Pro Thr Leu Pro Glu Gly Trp Thr Arg Lys Leu Val Gln Arg Lys Ser
1               5                   10                  15

Gly Arg Ser Ala Gly Lys Tyr Asp Val Leu Leu Ile Asn Pro Gln Gly
            20                  25                  30

Lys Ala Phe Arg Arg Lys Val Glu Leu Ile Ala Tyr Phe
        35                  40                  45
```

<210> 52
<211> 45
<212> PRT
<213> Artificial

<220>
<223> MBD variant of SEQ ID NO:2 (F275)

<400> 52

Pro Ala Leu Pro Pro Gly Trp Lys Lys Glu Glu Arg Ile Arg Lys Ser
1 5 10 15

Gly Leu Ser Ala Gly Lys Ser Asp Val Tyr Tyr Phe Ser Pro Ser Gly
20 25 30

Lys Lys Phe Arg Val Lys Pro Gln Leu Ala Arg Tyr Leu
35 40 45

<210> 53
<211> 45
<212> PRT
<213> Artificial

<220>
<223> MBD variant of SEQ ID NO:5 (L124F)

<400> 53

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Phe Ile Asn Pro Gln Gly
20 25 30

Lys Ala Phe Arg Ser Lys Val Glu Leu Ile Ala Tyr Phe
35 40 45

<210> 54
<211> 45
<212> PRT
<213> Artificial

<220>
<223> MBD variant of SEQ ID NO:5 (R133C)

<400> 54

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile Asn Pro Gln Gly
20 25 30

Lys Ala Phe Cys Ser Lys Val Glu Leu Ile Ala Tyr Phe
35 40 45

<210> 55
<211> 45
<212> PRT
<213> Artificial

<220>
<223> MBD variant of SEQ ID NO:5 (S134C)

<400> 55

Pro Thr Leu Pro Glu Gly Trp Thr Arg Lys Leu Lys Gln Arg Lys Ser
1 5 10 15

Gly Arg Ser Ala Gly Lys Tyr Asp Val Tyr Leu Ile Asn Pro Gln Gly
20 25 30

Lys Ala Phe Arg Cys Lys Val Glu Leu Ile Ala Tyr Phe
35 40 45

<210> 56
<211> 92
<212> PRT
<213> Artificial

<220>
<223> Variant of hMeCp2[90-181] (T158M)

<400> 56

Asp Arg Gly Pro Met Tyr Asp Asp Pro Thr Leu Pro Glu Gly Trp Thr
1 5 10 15

Arg Lys Leu Lys Gln Arg Lys Ser Gly Arg Ser Ala Gly Lys Tyr Asp
20 25 30

Val Tyr Leu Ile Asn Pro Gln Gly Lys Ala Phe Arg Ser Lys Val Glu
35 40 45

Leu Ile Ala Tyr Phe Glu Lys Val Gly Asp Thr Ser Leu Asp Pro Asn
50 55 60

Asp Phe Asp Phe Met Val Thr Gly Arg Gly Ser Pro Ser Arg Arg Glu
65 70 75 80

Gln Lys Pro Pro Lys Lys Pro Lys Ser Pro Lys Ala
85 90

<210> 57
<211> 80
<212> PRT
<213> Artificial

<220>
<223> hMBD2[146-225]

<400> 57

Ser Gly Lys Arg Met Asp Cys Pro Ala Leu Pro Pro Gly Trp Lys Lys
1 5 10 15

```
Glu Glu Val Ile Arg Lys Ser Gly Leu Ser Ala Gly Lys Ser Asp Val
            20                  25                  30

Tyr Tyr Phe Ser Pro Ser Gly Lys Lys Phe Arg Ser Lys Pro Gln Leu
        35                  40                  45

Ala Arg Tyr Leu Gly Asn Thr Val Asp Leu Ser Ser Phe Asp Phe Arg
    50                  55                  60

Thr Gly Lys Met Met Pro Ser Lys Leu Gln Lys Asn Lys Gln Arg Leu
65                  70                  75                  80


<210>  58
<211>  92
<212>  PRT
<213>  Artificial

<220>
<223>  hMeCp2[90-181]

<400>  58

Asp Arg Gly Pro Met Tyr Asp Asp Pro Thr Leu Pro Glu Gly Trp Thr
1               5                   10                  15

Arg Lys Leu Lys Gln Arg Lys Ser Gly Arg Ser Ala Gly Lys Tyr Asp
            20                  25                  30

Val Tyr Leu Ile Asn Pro Gln Gly Lys Ala Phe Arg Ser Lys Val Glu
        35                  40                  45

Leu Ile Ala Tyr Phe Glu Lys Val Gly Asp Thr Ser Leu Asp Pro Asn
    50                  55                  60

Asp Phe Asp Phe Thr Val Thr Gly Arg Gly Ser Pro Ser Arg Arg Glu
65                  70                  75                  80

Gln Lys Pro Pro Lys Lys Pro Lys Ser Pro Lys Ala
                85                  90


<210>  59
<211>  80
<212>  PRT
<213>  Artificial

<220>
<223>  hMBD1[2-81]

<400>  59

Ala Glu Asp Trp Leu Asp Cys Pro Ala Leu Gly Pro Gly Trp Lys Arg
```

1               5                   10                  15

Arg Glu Val Phe Arg Lys Ser Gly Ala Thr Cys Gly Arg Ser Asp Thr
            20                  25                  30

Tyr Tyr Gln Ser Pro Thr Gly Asp Arg Ile Arg Ser Lys Val Glu Leu
        35                  40                  45

Thr Arg Tyr Leu Gly Pro Ala Cys Asp Leu Thr Leu Phe Asp Phe Lys
    50                  55                  60

Gln Gly Ile Leu Cys Tyr Pro Ala Pro Lys Ala His Pro Val Ala Val
65                  70                  75                  80

<210> 60
<211> 80
<212> PRT
<213> Artificial

<220>
<223> hMBD3[2-81]

<400> 60

Glu Arg Lys Arg Trp Glu Cys Pro Ala Leu Pro Gln Gly Trp Glu Arg
1               5                   10                  15

Glu Glu Val Pro Arg Arg Ser Gly Leu Ser Ala Gly His Arg Asp Val
            20                  25                  30

Phe Tyr Tyr Ser Pro Ser Gly Lys Lys Phe Arg Ser Lys Pro Gln Leu
        35                  40                  45

Ala Arg Tyr Leu Gly Gly Ser Met Asp Leu Ser Thr Phe Asp Phe Arg
    50                  55                  60

Thr Gly Lys Met Leu Met Ser Lys Met Asn Lys Ser Arg Gln Arg Val
65                  70                  75                  80

<210> 61
<211> 92
<212> PRT
<213> Artificial

<220>
<223> hMBD4[76-167]

<400> 61

Ala Thr Ala Gly Thr Glu Cys Arg Lys Ser Val Pro Cys Gly Trp Glu
1               5                   10                  15

```
Arg Val Val Lys Gln Arg Leu Phe Gly Lys Thr Ala Gly Arg Phe Asp
            20                  25                  30

Val Tyr Phe Ile Ser Pro Gln Gly Leu Lys Phe Arg Ser Lys Ser Ser
        35                  40                  45

Leu Ala Asn Tyr Leu His Lys Asn Gly Glu Thr Ser Leu Lys Pro Glu
    50                  55                  60

Asp Phe Asp Phe Thr Val Leu Ser Lys Arg Gly Ile Lys Ser Arg Tyr
65                  70                  75                  80

Lys Asp Cys Ser Met Ala Ala Leu Thr Ser His Leu
                85                  90
```

## Description

### FIELD OF THE INVENTION

The present invention relates to an isolated Methyl-CpG binding domain (MBD) variant comprising an MBD core domain that has at least 60 % sequence homology relative to any one of SEQ ID Nos. 1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40 in SEQ ID NO:1. Furthermore, the invention relates to a conjugate comprising the isolated MBD variant and to the use of the isolated MBD variant or the conjugate for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC). Finally, the invention relates to specific methods for the determination of the methylation state of cytosine residues and/or oxidation state of the methyl group of 5-methylated cytosine residues as described above or for the enrichment of DNA molecules as described above.

### BACKGROUND OF THE INVENTION

Modifications of the canonical DNA nucleobases with small chemical groups such as a methyl or carboxy group, alter the physicochemical properties of the DNA major groove and in that way, the lead interface at which DNA-binding proteins such as transcription factors access the genetic information. This 'epigenetic' mechanism is used in many organisms to naturally control gene expression. As an example, 5-methylcytosine (mC), a pervasive modification of cytosine in mammalian genomes (4-5 mol% of all deoxycytidines), is decisively absent from activating regulatory elements such as gene promoters and enhancers.

In human and mouse, mC is almost exclusively limited to the deoxycytidine-phosphate-deoxyguanosine (CpG) dinucleotide context, with both deoxycytidines in the DNA double-strand being modified concurrently. The mC landscape is actively shaped throughout development and differentiation by DNA methyltransferases such as DNMT3a and DNMT3b, which establish mC at non-methylated CpGs, or DNMT1, which re-consolidates hemi-methylated CpGs after semi-conservative DNA replication. Furthermore, ten-eleven translocation (TET) dioxygenases iteratively oxidize mC to 5-hydroxymethyl-(hmC), 5-formyl- (fC), and 5-carboxycytosine (caC) (FIG. 1A), eventually promoting demethylation by base-excision repair.

The longevity of some of these oxidized mC species (collectively referred to as oxi-mC) at non-random sites in the mammalian genome, their effect on protein binding, DNA flexibility and their chemical reactivity, has established oxi-mC as additional epigenetic marks in genomes with biological functions that differ from the ones of mC.

In vitro, TET enzymes oxidize modified and hemi-modified CpGs in a non-processive manner, independent of the oxi-mC species on the complementary DNA strand. Thus, different mC and oxi-mC species likely co-exist at any CpG dinucleotide (FIG. 1B) in one of 15 conceivable, symmetric and asymmetric configurations (FIG. 1C). Yet, it is unknown which of these configurations exist *in vivo* and where they occur in the genome.

Direct sequencing of genomic DNA indicates that of all CpGs that contain hmC (6.2 mol%) only 21 % are symmetrically modified [1, 2]. Likewise, the mean difference of hmC or fC levels at CpGs in CpG-rich regions is 43-46 % (for mC this is 18 %) [3], and the genome-wide average correlation of oxi-mC levels within CpGs as low as 1-15 % (82 % for mC) [4].

Current technologies cannot resolve individual oxi-mC configurations because the read-out of the deoxycytidine modifications is based on bisulfite conversion and therefore binary. It is thus only possible to distinguish two oxi-mC species at once. Given a DNA molecule cannot be converted more than once, the fractional composition of each modified nucleobase must be inferred from the combination of different (bulk) sequencing experiments by probabilistic models. Consequently, the original oxi-mC configuration in single double-stranded DNA molecules cannot be determined.

Also, sequential affinity-enrichment with commercially available protein receptors against single oxi-mC [5, 6] is unsuitable to identify the oxi-mC composition at CpGs as neighboring modifications on the same DNA fragment cannot be discriminated. Antibodies, for example, have the additional drawback that they do not provide strand-selectivity. Thus, the lack of suitable binders to capture defined oxi-mC configuration at CpGs impedes further understanding of their biological function in the mammalian genome.

### SUMMARY OF THE INVENTION

Surprisingly, the inventors found specific engineered Methyl-CpG binding domains (MBD) that preferably provide differential interactions with different mC and/or oxi-mC configurations at (single) CpGs in a DNA molecule. Based on these findings, the inventors created molecular probes on the basis of these engineered Methyl-CpG binding domains which preferably resolve the precise configuration of oxi-mC species across the DNA double-strand, more preferably at the single-molecule level. These molecular probes or engineered Methyl-CpG binding domains can find application in diverse analytical formats, ranging from affinity enrichment over imaging, flow cytometry, improved direct high-throughput sequencing methods and others. For example, they can enable affinity enrichment assays with MBD-functionalized capture beads that enable retrieving double-stranded DNA molecules with defined oxi-mC configurations from complex genomic DNA samples (FIG. 2). In another example, they can be used for mapping CpGs with distinct configurations, and ultimately allow correlating such genome-wide maps with known (epi-)genetic features to reveal biological functions.

Therefore, in a first aspect, the invention relates to an isolated Methyl-CpG binding domain (MBD) variant, comprising an MBD core domain that has at least 60 % sequence homology relative to any one of SEQ ID Nos. 1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26, 37, in SEQ ID NO:1.

The inventors have found that these positions are involved with base recognition and binding and/or are located in the three-dimensional structure such as to contact the cytosine base of the bound DNA molecule.

In various embodiments, the variant comprises said at least one amino acid substitution relative to the corresponding wildtype MBD in at least one of the positions corresponding to positions 12, 19, 21, 22, 23, 25, 26, 27, 35, 36, 37, 38, and 39 in SEQ ID NO:1.

In various embodiments, the variant comprises said at least one amino acid substitution relative to the corresponding wildtype MBD in at least one of the positions corresponding to positions 12, 25, 26, 27, 36 and 37 in SEQ ID NO:1.

In one embodiment, the variant comprises said at least one amino acid substitution relative to the corresponding wildtype MBD in at least one of the positions corresponding to positions 12, 25, 26, 37 in SEQ ID NO:1.

In various embodiments, the variant comprises two or more of the above-mentioned amino acid substitutions, such as 3, 4, 5, 6, 7, 8, 9, 10, 11, 12, 13, 14, 15 or all 16. In various embodiments, the variant comprises at least 2, 3 or 4 amino acid substitutions. These are preferably selected from substitutions in positions 12, 25, 26 and 37. In embodiments, where the variant comprises more than 4 substitutions, the 5$^{th}$ and subsequent substitutions may occur in positions 19, 21, 22, 23, 27, 35, 38 and 39. In embodiments, where the variant comprises more than 12 substitutions, the additional substitutions may be selected from those at positions 14, 24, 36, and 40. In some embodiments, the variant does not comprise any other modifications, in particular no other amino acid substitutions, outside of the positions listed herein.

In various embodiments, said at least one amino acid substitution is selected from 12S, 12T, 12A, 12V, 12R, 25I, 25T, 25A, 25C, 25L, 26T, 26S, 26F, 26L, 27F, 36C, 37N, 37K, 37Q, 37R, 37V and 37C, preferably 12S, 12T, 12A, 12V, 12R, 25I, 25T, 25A, 25C, 25L, 26T, 26S, 26F, 26L, 37N, 37K, 37Q, 37R and 37V, more preferably 12S, 12T, 25I, 25T, 25A, 26T, 37N and 37K using the positional numbering of SEQ ID NO:1.

Preferably, the MBD core domain which is comprised in the isolated MBD variant has at least 65, 70, 75 or 80 % sequence homology or sequence identity to any one of SEQ ID Nos. 1-45 over its entire length.

In various embodiments, the MBD core domain comprises any one or more of the amino acids 14R/K, 24D/E, 36R/K and 40E/Q/D using the positional numbering of SEQ ID NO:1. These amino acids typically correspond to those at the same position in the wildtype MBD and are in some embodiments invariable.

In various other embodiments, the MBD core domain comprises any one or more of the amino acids 14R/K, 24D/E, 36R/K and 40E/Q/D/S using the positional numbering of SEQ ID NO:1. These amino acids typically correspond to those at the same position in the wildtype MBD and are in some embodiments invariable.

In various other embodiments, the MBD core domain comprises any one or more of the amino acids 14R/K/T, 24D/E/H, 36R/E/S/K and 40E/Q/S, preferably any one or more of the amino acids 14R, 24D, 36R and 40E/Q/S, using the positional numbering of SEQ ID NO:1. These amino acids typically correspond to those at the same position in the wildtype MBD and are in some embodiments invariable.

In various embodiments, the MBD core domain comprises any one or more of the amino acids 1P/K, 3L/V, 6G/D, 7W/F, 8R/Q/K/E, 9R/K, 17G, 27Y/F/L, 30P, 32G, 44Y/F and 45L/I using the positional numbering of SEQ ID NO:1. These amino acids typically correspond to those at the same position in the wildtype MBD and are in some embodiments invariable.

In various other embodiments, the MBD core domain comprises any one or more of the amino acids 1P/K, 3L/V, 6G/D, 7W/F, 8R/Q/K/E/T, 9R/K, 17G, 27Y/F/L, 30P, 32G, 44Y/F and 45L/I/F using the positional numbering of SEQ ID NO:1. These amino acids typically correspond to those at the same position in the wildtype MBD and are in some embodiments invariable.

In various other embodiments, the MBD core domain comprises any one or more of the amino acids 1P/K/S/T/W/A/L/I/F/V, 3L/V/T/I, 6G/D/H, 7W/F, 8R/Q/K/E/T, 9R/K/M, 17G/A/Y/S/N/H, 27Y/F/L, 30P, 32G, 44Y/F/A and 45L/I/F/V, preferably any one or more of the amino acids 1P/K, 3L/V, 6G, 7W, 8K/E/T, 9R/K, 17G, 27Y/F/L, 30P, 32G, 44Y, 45L/F, using the positional numbering of SEQ ID NO:1. These amino acids typically correspond to those at the same position in the wildtype MBD and are in some embodiments invariable.

In various embodiments, the MBD core domain comprises any one or more of the amino acids 1P/K, 2A/S/T, 3L,V, 4G/P, 5P/Q/C/E, 6G/D, 7W/F, 8R/Q/K/E, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N, 30P, 31T/S/Q, 32G, 33D/K/L, 34R/K/A, 35I/F, 36R/K, 37S, 38K, 39V/P/S, 40E/Q/D, 41L, 42T/A/I, 43R/N/A, 44Y/F/V, 45L/I using the positional numbering of SEQ ID NO:1. These amino acids typically correspond to those at the same position in the wildtype MBD and are in some embodiments invariable.

In various embodiments, the MBD core domain comprises any one or more of the amino acids 1P/K, 2A/S/T, 3L,V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N, 30P, 31T/S/Q, 32G, 33D/K/L, 34R/K/A, 35I/F, 36R/K, 37S, 38K, 39V/P/S, 40E/Q/D/S, 41L, 42T/A/I, 43R/N/A, 44Y/F/V, 45L/I/F using the positional numbering of SEQ ID NO:1. These amino acids typically correspond to those at the same position in the wildtype MBD and are in some embodiments invariable.

In various embodiments, the MBD core domain comprises any one or more of the amino acids 1P/K/S/T/W/A/L/I/F/V, 2A/S/T/P/L/G/K/R, 3L,V/T/I, 4G/P/A/Q/E/L/K/R, 5P/Q/C/E/N/K/A/L/R/H/Y, 6G/D/H, 7W/F, 8R/Q/K/E/T, 9R/K/M, 10R/E/V/K/Q/S/M, 11E/V/L/N/T/H, 12V/K/S/A/I/C/R/G, 13F/I/P/Q/T/V/L/R/K, 14R/K/T, 15K/R/L/Q/S/N, 16F/S/L/T/I/D/G/V, 17G/A/Y/S/N/H, 18A/L/K/R/P/S, 19T/S/G/A/H/R, 20C/A/F/D/R/G/K/S, 21G/I/V/W/L/M/S, 22R/K/H/Q/G/A/S, 23S/R/F/Y/T/G/V/M/L, 24D/E/H, 25T/V/I/A, 26Y/F/I/S/W/A/N, 27Y/F/L, 28Q/F/Y/I/R/K, 29S/N/H/A/T/G, 30P, 31T/S/Q/E/N/A/C, 32G, 33D/K/L/E/R, 34R/K/A/C/S/N, 35I/F/L/M, 36R/E/S/K, 37S/T/Q/N, 38K/R/Y/F/M/V, 39V/P/S/R/I/N/Q/E/A, 40E/Q/S, 41L/V/I, 42T/A/I/M/V/E/F/Q, 43R/N/A/K/H, 44Y/F/A, 45L/I/F/V, preferably any one or more of the amino acids 1P/K, 2A/S/T, 3L/V, 4G/P, 5P/Q/C/E, 6G, 7W, 8K/E/T, 9R/K, 10R/E/V/K, 11E/V/L, 12V/K, 13F/I/P/Q, 14R, 15K/R/L, 16S/F, 17G, 18A/L/K/R, 19T/S, 20C/A, 21G, 22R/K/H, 23S/R/F/Y, 24D, 25T/V, 26Y/F, 27Y/F/L, 28Q/F/Y/I, 29S/N, 30P, 31T/S/Q, 32G, 33D/K/L, 34R/K/A, 35I/F, 36R, 37S, 38K, 39V/P/S, 40E/Q/S, 41L, 42T/A/I, 43R/N/A, 44Y, 45L/F, using the positional numbering of SEQ ID NO:1. These amino acids typically correspond to those at the same position in the wildtype MBD and are in some embodiments invariable.

If in any of the above embodiments, it is preferred to keep the amino acids at positions that have not been listed for substitution herein.

Preferably, the isolated MBD variant has, relative to the corresponding wildtype MBD, an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

In various embodiments, said variant has an altered binding affinity, preferably increased affinity, for CpG dinucleotides and their complement in which

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC).

In various embodiments, said variant has differential binding affinity for any two CpG dinucleotides and their complement selected from the following oxidized 5-methylated cytosine configurations:

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);

(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or (l) both cytosine bases are 5-carboxylated (caC).

Preferably, the isolated MBD variant comprises, consists essentially of or consists of any one of the amino acid sequences set forth in SEQ ID Nos. 46 to 52 and/or 53 to 55.

In a second aspect, the invention relates to a conjugate comprising the isolated MBD variant according to the invention.

Preferably, the conjugate further comprises an enzyme, preferably a nuclease or methyltransferase, or a detectable label, preferably a fluorophore.

In another aspect, the invention relates to a use of the isolated MBD variant as described herein or the conjugate as described herein for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

In still another aspect, the invention relates to a method for the determination of the methylation state of cytosine residues and/or oxidation state of the methyl group of 5-methylated cytosine residues in a CpG dinucleotide of interest and its complement in a DNA molecule, said method comprising

(a) providing a molecular probe comprising a Methyl-CpG binding domain (MBD) that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

In various embodiments of the method, the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.

In a further aspect, the invention relates to a method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising

(a) providing a molecular probe comprising a Methyl-CpG binding domain (MBD) that binds to the region of the DNA molecule comprising said CpG dinucleotide of interest and differentially binds to different methylated cytosine and/or oxidized 5-methylcytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

In various embodiments of this method, the molecular probe is immobilized on a substrate.

Preferably, the molecular probe comprises an affinity ligand that allows immobilization of the molecular probe on a substrate.

The enrichment step of the method may comprise separating the complexes of the DNA molecules with the immobilized molecular probe from the non-complexed DNA molecules, the enrichment optionally including chromatography, centrifugation or magnetic bead separation.

In various embodiments of the methods described herein, the molecular probe comprises a variant MBD that comprises at least one amino acid substitution relative to the respective wildtype MBD, preferably an MBD variant as defined for the variant according to the invention.

In various embodiments of the methods according to the invention, the difference in binding affinity of the molecular probe is an increased binding affinity for an oxidized state of 5-methylated cytosine, in particular 5-hydroxymethylcytosine, relative to the non-oxidized 5-methylated cytosine.

In various embodiments of the methods according to the invention, the difference in binding affinity of the molecular probe is an increased binding affinity for a 5-methylated state of cytosine relative to the non-methylated cytosine.

The invention will be better understood with reference to the detailed description when considered in conjunction with the non-limiting examples and the accompanying figures.

## BRIEF DESCRIPTION OF THE DRAWINGS

**FIG. 1:** A) Chemical structures and color-code of the relevant nucleobases. B) Scheme of a (abridged subsequence of a DNA double-strand containing a CpG dinucleotide; the different modified cytosine species are indicated by color. C) Possible combinations of cytosine modifications at CpG dinucleotides.

**FIG. 2:** A possible application of the invention is the isolation of short DNA duplexes ('fragments'), which can be derived e.g. from genomic DNA, that contain a CpG dinucleotide with a defined configuration of cytosine modifications. To this end, the MBD is immobilized on functionalized capture beads for affinity enrichment. The captured DNA fragments are eluted from the beads and can be sequenced by next-generation sequencing so that the genomic positions of the configuration states can be identified.

**FIG. 3:** A) Model of an MBD bound to a DNA double-strand containing a fully methylated CpG dinucleotide (mC/mC). The interaction with each DNA strand involves (but is not limited to) two distinct structures within the MBD, helix $\alpha_1$ and loop L1; adapted from PDB:1ig4. B) Residues interacting with the nucleobases in proximity to the CpG dinucleotide; the numbering is according to the positional numbering of SEQ ID NO:1; bold sites indicate amino acids, which can be preferably substituted relative to the wildtype sequence.

**FIG. 4:** Overview of amino acid sequences (MBD core domain) of SEQ ID Nos. 1-45.

**FIG. 5:** Overview of amino acid sequences (MBD core domain) of SEQ ID Nos. 46-52 (bold sites indicate substituted amino acids relative to the corresponding wildtype sequence).

**FIG. 6:** A) Probe-wise determination of binding affinity using fluorescently labelled DNA duplexes that contain a single CpG dinucleotide with one of the configurations of FIG. 1; these are incubated with an isolated and purified MBD protein or an isolated and purified MBD protein fused to a 'tag' (as specified herein) or to an isolated population of different E. *coli* clones that each present a single species of MBD protein on their cell surface. Typically, relative affinity of isolated and purified MBD proteins is determined with an electrophoretic mobility shift assay (EMSA), whereas the relative affinity of MBD proteins displayed on the cell surface is determined using a fluorescence-activated cell sorting (FACS) on a flow cytometer. B) Assay validation for an mC/mC-binding wildtype MBD protein containing SEQ ID NO:2 (hMBD2[148-225] on EMSA and FACS (not all probes shown)). C) Assay validation for a non-binding wildtype MBD protein containing SEQ ID NO:3 (hMBD3[2-81]) on EMSA and FACS (not all probes shown). D) Systematic profiling of a wildtype MBD protein containing SEQ ID NO:2 (hMBD2[148-225]) at a high and a low protein concentration on EMSA. E) The same MBD protein displayed on a cell surface after induction of MBD expression with 50 $\mu$M isopropyl $\beta$-$_D$-1-thiogatactopyranoside for 60 min. Population mean fluorescence intensities of three independent replicates are shown.

**FIG. 7:** A) Selectivity of the hMeCp2[90-181]-derived ('wildtype') purified MBD proteins F271, F272, F273, and F274 on EMSA using mC/mC, mC/caC and caC/caC probes. B) Full EMSA-profile of the purified proteins F105, F106, F271, F272, F273, F274 and the hMBD2[148-225]-derived protein F275 on EMSA. Arrows indicate distinct differences from wildtype behavior. F105, F271-F274 were expressed as MBP-fusion protein.

**FIG. 8:** Quantification of the selectivity based on EMSA as shown in FIG. 7B as the fraction of bound DNA duplex at 1,024 nM MBD protein. The main selectivity within the group of mC/mC, mC/hmC, mC/fC, and mC/caC are indicated by shading. Arrows indicate distinct differences from wildtype behavior.

**FIG. 9:** Determination of the dissociation constant Kd for the hMeCp2 [90-181]-derived ('wildtype') purified MBD protein F106 (SEQ ID NO:47) for mC/hmC and mC/mC using EMSA; and validation of binding selectivity in a FACS assay with surface-displayed F106 and differentially labeled DNA duplexes at equimolar ratio.

**FIG. 10:** Unpurified candidates (mixtures of surface-displayed MBD domains) for selective recognition of the oxidized mC CpG configurations as indicated. The candidates are derived from codon-degenerated scaffolds comprising SEQ ID Nos. 2, 3, or 5. Relative selectivity was quantified in separate reactions with the same fluorophore-streptavidin conjugate using either a dsDNA probe containing the indicated configuration or an equimolar mixture of all other probes.

**FIG. 11:** Representative single measurements of the fraction of bound labelled DNA duplex are summarized (mean) as bar graphs at high MBD protein concentration. Error bars indicate standard error of the mean with two-sided Student's t-test against 'wildtype' MeCp2[90-181] (ns: p in (0.1, 1], . (0.05, 0.1], * (0.01, 0.05], ** (0.001, 0.01], *** [0, 0.001]) for the variants L124F (SEQ ID NO:53), R133C (SEQ ID NO:54), S134C (SEQ ID NO:55) and T158M (SEQ ID NO:56) of human MeCp2[90-181] (SEQ ID NO:58).

**FIG. 12:** A) Overview of amino acid sequences (MBD core domain) of SEQ ID Nos. 53 to 55; B) T158M variant of human MeCp2[90-181] (SEQ ID NO:56) (grey-shaded amino acids indicate the MBD core domain and bold amino acids indicate the amino acid substitution).

**FIG. 13:** Wildtype MBD protein sequences of human MBD2[146-225] (SEQ ID NO:57), human MeCp2[90-181] (SEQ ID NO:58), human MBD1[2-81] (SEQ ID NO:59), human MBD3[2-81] (SEQ ID NO:60), human MBD4[76-167] (SEQ ID NO:61) (grey-shaded amino acids indicate the MBD core domain).

## DETAILED DESCRIPTION OF THE INVENTION

The terms "one or more" or "at least one", as interchangeably used herein, relate to at least 1, or at least 2, 3, 4, 5, 6, 7, 8, 9, 10, 11, 12, 13, 14, 15, 20, 25 or a plurality of species, e.g. at least one amino acid substitution. In this connection, the term "plurality" means more than one, preferably 2 or more, such as up to 1000.

Numeric values specified without decimal places here refer to the full value specified with one decimal place, i.e. for example, 99 % means 99.0 %, unless otherwise defined.

The terms "approx." and "about", in connection with a numerical value, refer to a variance of ±10 %, preferably ±5 %, more preferably ±2 %, more preferably ±1 %, more preferably ±0.1 %, and most preferably less than ±0.1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

"Substantially", for example in "substantially purified", typically means that the given amount or property is dominant compared to other amounts or properties. With respect to purification, this means that little amounts of impurities, i.e. the to-be-separated species, can still be present. Preferably, these impurities are comprised in amounts of less than 10 wt.-%, more preferably less than 5 wt.-%, more preferably less than 1 wt.-%, more preferably less than 0.1 wt. %, more preferably less than 0.01 wt.-%, more preferably less than 0.001 wt.-%, most preferably these impurities are not present, if not explicitly stated otherwise. With respect to properties, it means that the original properties/functionalities are retained to a considerable extent, for example by at least 80% with respect to functionalities and activities.

The present invention relates to an isolated Methyl-CpG binding domain (MBD) variant, comprising an MBD core domain that has at least 60 % sequence homology relative to any one of SEQ ID Nos.1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26, 37, in SEQ ID NO:1.

In various embodiments, the isolated Methyl-CpG binding domain (MBD) variant comprises an MBD core domain that has at least 60 % sequence homology relative to any one of SEQ ID Nos.1-5, preferably SEQ ID NO:2 or 5, and comprises at least one amino acid substitution relative to the corresponding wildtype MBD in at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26, 37, in SEQ ID NO:1.

As used herein, the term "Methyl-CpG binding domain (MBD) variant" refers to a Methyl-CpG binding domain or a segment or fragment thereof that varies in its sequence compared to a naturally occuring version of the same molecule. In various embodiments, this means that its sequence has one or more amino acids added, deleted, substituted or otherwise chemically modified in comparison to the corresponding wildtype MBD or the corresponding segment or fragment thereof. It is however understood that such variants substantially retain the same properties as the wildtype MBD to which they are compared, in particular such that they still have the characteristic functionality and activity of an MBD.

Typically, the modified MBD (variant) is "isolated" from the cell in which it was generated, using standard techniques known to the person skilled in the art. The term can also apply to a variant, which has been substantially purified from other components, which naturally accompany the variant, e.g., proteins, RNA or DNA which naturally accompany it in the cell.

However, it is also included in the scope of the present invention that the variant can be displayed on the surface of a cell, preferably on the surface of *E. coli,* using standard techniques known to the person skilled in the art (e.g. AIDA surface display). The term therefore includes, for example, a recombinant MBD or a segment or fragment thereof, which is encoded by a nucleic acid incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences.

In some embodiments, the variant is based on the Methyl-CpG binding domain of an MBD or MeCp2 protein, which is recombinantly produced, preferably in *E. coli,* and subsequently either isolated from the cell for further use or displayed on the cell surface, preferably on the cell surface of *E. coli.*

The term "MBD core domain" refers to a domain comprised in the amino acid sequence of the MBD wildtype or variant, which is typically necessary to enable the binding of MBD with a DNA molecule comprising a CpG dinucleotide, if not explicitly stated otherwise. The core domain is typically about 45 amino acids in length. In various embodiments, the core domain is 42 to 46 amino acids in length, preferably 42, 43, 44, 45 or 46 amino acids in length.

Preferably, said MBD variant has relative to the wildtype MBD an altered affinity, preferably an increased or decreased affinity, for a DNA molecule comprising a CpG dinucleotide of interest and its complement in which at least one cytosine nucleobase in the CpG dinucleotide of interest is modified, wherein the cytosine nucleobase is selected from the group of cytosine (C), 5-methylcytosine (mC), 5-hydroxymethylcytosine (hmC), 5-formylcytosine (fC), and 5-carboxylcytosine (caC), preferably the cytosine nucleobase in the CpG dinucleotide of interest is modified and is selected from the group of 5-methylcytosine (mC), 5-hydroxymethylcytosine (hmC), 5-formylcytosine (fC), and 5-carboxylcytosine (caC), more preferably from the group of 5-hydroxymethylcytosine (hmC), 5-formylcytosine (fC), and 5-carboxylcytosine (caC).

"Altered affinity", as used herein, means that the affinity if tested in a suitable assay, such as calorimetry, surface plasmon resonance, etc, detectably differs compared to the corresponding wildtype protein. "Detectably differs" thus means that the difference falls outside the error margins of the measurement. In various embodiments, such an alteration or difference in affinity means a change by at least 10 %, at least 20 %, at least 50 % or more. In various embodiments, the change is at least 2-fold, more preferably at least 5-fold or most preferably at least one order of magnitude (10-fold). This applies in two directions, i.e. the affinity may be double the original value or half the original value, etc.

The term "CpG-dinucleotide" describes a part of a DNA molecule in which two nucleotides containing the nucleobases cytosine and guanine are linked to each other. Typically, the CpG-dinucleotide contains or consists of the unit deoxycytidine-phosphate-deoxyguanosine (in 5'-3' direction). In general, without being limited to this, the presence and level of the CpG-dinucleotide in a DNA molecule is organism- and region-dependent. CpG sites occur with high frequency in genomic regions called CpG islands (or CG islands) with the cytosine often being subject to methylation. In the target nucleic acid sequences, i.e. those sequences targeted by the MBD, the nucleobase cytosine can thus be modified and selected from the group of 5-methylcytosine (5mC), 5-hydroxymethylcytosine (hmC), 5-formylcytosine (fC), and 5-carboxylcytosine (caC). It is an object of the invention to design MBD variants that can distinguish between those different variants.

The term "sequence identity" as used herein refers to polypeptides, peptides or proteins or segments or fragments thereof that share identical amino acids at corresponding positions or nucleic acids sharing identical nucleotides at corresponding positions. The broader concept of "sequence homology" also takes conserved amino acid exchanges into account in the case of amino acid sequences, i.e. amino acids having similar chemical activity, since they usually perform similar chemical activities within the protein. Under the concept of sequence homology, conservative amino acid changes are thus not counted as changes. Conservative replacements means a change within the following groups of amino acids:

Hydrophobic: M, A, V, L, I
Neutral/Hydrophilic: S, C, T, N, Q
Acidic: D, E
Basic: H, K, R
Residues that influence chain orientation: G, P
Aromatic: W, Y, F

The determination of percent sequence homology or percent sequence identity described herein between two amino acid or nucleotide sequences can be accomplished using a mathematical algorithm. For example, a mathematical algorithm useful for comparing two sequences is the algorithm incorporated into the BLASTN and BLASTX programs and can be accessed, for example, at the National Center for Biotechnology Information (NCBI) world wide web site having the universal resource locator "www.ncbi.nlm.nih.gov/BLAST". Blast nucleotide searches can be performed with BLASTN program, whereas BLAST protein searches can be performed with BLASTX program or the NCBI "blastp" program.

Identity or homology information can be provided regarding whole polypeptides, peptides, proteins or genes or only regarding individual regions. However, identity or homology information in the present application relates to the entire length of the particular nucleic acid or amino acid sequence indicated. For example, percent homology is based on the entire amino acid sequence of the MBD core domain according to any one of SEQ ID Nos. 1-45, preferably to any one of SEQ ID Nos. 1-32, more preferably to anyone of SEQ ID Nos. 1-10, most preferably to anyone of SEQ ID Nos. 1-5, in particular according to any one of SEQ ID NO: 1, 2, 3, 4, 5, 6, 7, 8, 9, or 10.

According to the present invention, the feature "at least 60 % sequence homology" includes every variation, in particular every amino acid substitution, in the MBD core domain of the Methyl-CpG-binding domain variant which is/are present relative to any one of SEQ ID Nos. 1-45, preferably to any one of SEQ ID Nos. 1-32, more preferably to any one of SEQ ID Nos. 1-10, most preferably to any one of SEQ ID Nos. 1-5. "60% homology" thus means that 60% of all amino acids present at the corresponding positions in the variant are either substituted such that they are still homologous or are unaltered. In preferred embodiments, the MBD core domain of the MBD variant has at least 65, 70, 75 or 80 % sequence homology or sequence identity to any one of SEQ ID Nos.1-45, preferably to any one of SEQ ID Nos.1-32, more preferably to any one of SEQ ID NO:1, 2, 3, 4, 5, 6, 7, 8, 9, or 10. In various embodiments, the "60% homology" are "60% identity".

In various embodiments, the sequence homology or identity is at least 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, or 97 %.

The variants comprise at least one amino acid substitution relative to the corresponding wildtype MBD in at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26 and 37 according to SEQ ID NO:1. SEQ ID NO:1 is used as a reference here to allow positional numbering. The corresponding positions to the positions in SEQ ID NO:1 can be determined by an alignment, as explained above. In various embodiments, the variants described herein comprise only 1 amino acid substitution relative to the template sequence. In various other embodiments, the variants comprise two or more, 3 or more, 4 or more, 5 or more, 6 or more, 7 or more, 8 or more, 9 or more, 10 or more, 11 or more, 12 or more, 13 or more, 14 or more, 15 or 16 substitutions. These substitutions are preferably selected from substitutions in the positions listed above, with the target amino acids being any amino acid with the exception of the amino acid occurring naturally at this position, preferably being those described herein.

In various preferred embodiments, said at least one amino acid substitution relative to the corresponding wildtype MBD is selected from 12S, 12T, 12A, 12V, 12R, 25I, 25T, 25A, 25C, 25L, 26T, 26S, 26F, 26L, 27F, 36C, 37N, 37K, 37Q, 37R, 37V and 37C, preferably 12S, 12T, 12A, 12V, 12R, 25I, 25T, 25A, 25C, 25L, 26T, 26S, 26F, 26L, 37N, 37K, 37Q, 37R and 37V, more preferably 12S, 12T, 25I, 25T, 25A, 26T, 37N and 37K using the positional numbering of SEQ ID NO:1.

The term "positional numbering" refers to the numbering and arrangement of the present amino acids of the MBD core domain of the MBD wildtype construct according to SEQ ID Nos. 1, if not explicitly stated otherwise. SEQ ID Nos. 2-27 and 29-30 have the same positional numbering as SEQ ID NO:1. In contrast, SEQ ID NO: 28 comprises an insertion at position 20 and SEQ ID Nos. 31-45 contain amino acid deletions (FIG. 4). "Positions corresponding to positions [...] in SEQ ID NO:1" thus refers to those positions that correspond to the respective numbered position in SEQ ID NO:1 in an alignment.

For example, the amino acid sequence set forth in SEQ ID NO:2, can be modified with at least one of the following amino acid substitutions: V12S, V12T, V12R, V25I, V25T, V25A, Y26T, S37N, S37K, S37V. For example, "V12S" means that the amino acid valine which is present at position 12 in the wildtype MBD core domain of the human MBD2 is substituted by (replaced with) a serine.

In a preferred embodiment, the amino acid sequence set forth in SEQ ID NO:2 is modified with the following amino acid substitutions: V12R and S37V.

In another preferred embodiment, the amino acid sequence set forth in SEQ ID NO:5 is modified with one of the following amino acid substitution(s): i) L27F; ii) R36C, iii) S37C, iv) K12T, V25T, Y26T, S37K; iv) K12T, V25A, S37N; v) K25C, Y26S, S37Q; vi) K12A, V25C, Y26F, S37R; vii) K25L, Y26T, S37R; viii) K12V, Y26L, S37R.

In various embodiments, the isolated MBD variant comprises one of the following amino acid substitutions or amino acid substitution combinations, preferably in its MBD core domain:

i) 12S; ii) 12T; iii) 25I; iv) 25T; v) 26T; vi) 37N; vii) 37K; viii) 12S, 25I; ix) 12S, 25T; x) 12S, 26T; xi)12S, 37N; xii) 12S, 37K; xiii) 12T, 25I; xiv) 12T, 25T; xv) 12T, 26T; xvi) 12T, 37N; xvii) 12T, 37K; xviii) 25I, 26T; xix) 25I, 37N; xx) 25I, 37K; xxi) 25T, 26T; xxii) 25T, 37N; xxiii) 25T, 37K; xxiv) 26T, 37N; xxv) 26T, 37K; xxvi) 12S, 25I, 26T; xxvii) 12S, 25I, 37N; xxviii) 12S, 25I, 37K; xxix) 12S, 25T, 26T; xxx) 12S, 25T, 37N; xxxi) 12S, 25T, 37K; xxxii) 12S, 26T, 37N; xxxiii) 12S, 26T, 37K; xxxiv) 12T, 25I, 26T; xxxv) 12T, 25I, 37N; xxxvi) 12T, 25I, 37K; xxxvii) 12T, 25T, 26T; xxxviii) 12T, 25T, 37N, xxxix) 12T, 25T, 37K; xl) 12T, 26T, 37N; xli) 12T, 26T, 37K; xlii) 25I, 26T, 37N; xliii) 25I, 26T, 37 K; xliv) 25T, 26T, 37N; xlv) 25T, 26T, 37K; xlvi) 12S, 25I, 26T, 37N; xlvii) 12S, 25I, 26T, 37K; xlviii) 12S, 25T, 26T, 37N; xlix) 12S, 25T, 26T, 37K; l) 12T, 25I, 26T, 37N; li) 12T, 25I, 26T, 37K; lii) 12T, 25T, 26T, 37N; or liii) 12T, 25T, 26T, 37K, preferably relative to the corresponding wildtype MBD according to any one of SEQ ID Nos. 1-45, wherein the positional numbering is according to SEQ ID NO:1.

In various embodiments, the isolated MBD variant comprises one of the following amino acid substitutions or amino acid substitution combinations, preferably in its MBD core domain:

i) 12T, 25T, 26T, 37K; ii) 12T, 25A, 37N; iii) 25C, 26S, 37Q; iv) 12A, 25C, 26F, 37R; v) 25L, 26T, 37R; vi) 12V, 26L, 37R; vii) 12R, 37V, viii) 27F; ix) 36C; x) 37C; relative to the corresponding wildtype MBD according to any one of SEQ ID Nos. 1-45, preferably to any one of SEQ ID Nos. 1, 2, 3, 4 or 5, more preferably to any one of SEQ ID NO:2 or 5, wherein the positional numbering is according to SEQ ID NO:1.

In various embodiments, to ensure MBD functionality, some of the original amino acid positions are not altered but maintained. In the following, some of these consensus sequences are disclosed.

In various embodiments, the MBD core domain of the isolated MBD variant comprises any one or more of the amino acids 14R/K, 24D/E, 36R/K and 40E/Q/D, optionally any one or more of the amino acids 14R/K, 24D/E, 27Y/F/L, 36R/K and 40E/Q/D/S, using the positional numbering of SEQ ID NO:1. In some embodiments, one or more of the amino acids 14R/K, 24D/E, 36R/K and 40E/Q/D/S, optionally any one or more of the amino acids 14R/K, 24D/E, 27Y/F/L, 36R/K and 40E/Q/D/S, are present in addition to the at least one amino acid substitution selected from 12S, 12T, 25I, 25T, 25A, 26T, 37N and 37K using the positional numbering of SEQ ID NO:1. These amino acids at positions 14, 24, 27, 36 and 40 using the positional numbering according to SEQ ID NO:1 correspond to the amino acids at the corresponding positions in the wildtype MBD core domain and are thus in some embodiments unaltered, i.e. not substituted. This can help to ensure MBD functionality.

In various embodiments, the isolated MBD variant comprises any one or more of the amino acids 1P/K, 3L/V, 6G/D, 7W/F, 8R/Q/K/E/T, 9R/K, 17G, 27Y/F/L, 30P, 32G, 44Y/F and 45L/I/F, preferably 1P/K, 2A/S/T, 3L,V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N, 30P, 31T/S/Q, 32G, 33D/K/L, 34R/K/A, 35I/F, 36R/K, 37S, 38K, 39V/P/S, 40E/Q/D/S, 41L, 42T/A/I, 43R/N/A, 44Y/F/V, 45L/I/F, preferably using the positional numbering of SEQ ID NO:1. This is the full consensus sequence of the MBDs disclosed herein. It is understood that the feature that the MBD variants comprise an amino acid substitution at at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26 and 37, according to SEQ ID NO:1 means that the above amino acid in the consensus sequence is replaced by another amino acid, which is typically not a consensus amino acid. This means, for example, that if the amino acid substitution is in position 26, the target amino acid is preferably not F (including if the starting amino acid is Y) or Y (including if the starting amino acid is F). In various embodiments, the target amino acid of any of the substitutions according to the invention is thus not any one listed above as a consensus amino acid.

In various embodiments, the MBD core domain of the isolated MBD variant comprises at least one amino acid substitution selected from 12S, 12T, 12A, 12V, 12R, 25I, 25T, 25A, 25C, 25L, 26T, 26S, 26F, 26L, 27F, 36C, 37N, 37Q, 37R, 37V, 37C and 37K, preferably selected from 12T, 12A, 12V, 12R, 25T, 25A, 25C, 25L, 26T, 26S, 26F, 26T, 26L, 37K, 37N, 37Q, 37R and 37V or from 27F, 36C and 37C, while retaining consensus amino acids according to the above list in all other positions. Typically, these MBD variants comprise an MBD core domain that has preferably at least 65, 70, 75 or 80 %, sequence homology or identity relative to any one of SEQ ID Nos.1-45, preferably to any one of SEQ ID Nos. 1, 2, 3, 4, or 5.

Again, the above listed amino acids for positions 1-45 correspond to the amino acids at the corresponding positions in the wildtype MBD core domain, i.e. form a consensus sequence of the wildtype MBD core domain, and are thus in some embodiments unaltered, i.e. not substituted. It is understood that when some of these positions overlap with those that are disclosed herein as optionally being substituted, such as positions 12, 14, 19, 25, etc., that the substitutions prevail. In all embodiments, it is, however, preferred that the non-substituted positions are occupied by amino acids as listed above. This means, for example, that if position 25 is not substituted, it is preferably T or V, which typically corresponds to the respective wildtype sequence.

In various embodiments, the polypeptide comprising the MBD domain may comprise at least one amino acid substitution in the amino acid sequence N- or C-terminal to the MBD core domain, preferably C-terminal to the MBD core domain, in particular in position 158 of hMeCp2[90-181] (SEQ ID NO:58) (16 amino acids C-terminal to amino acid 45 of the MBD core domain of SEQ ID NO:5 (position 61). Most preferably, the amino acid sequence of variant T158M comprises or consists of the amino acid sequence set forth in SEQ ID NO:56. In such embodiments, the MBD core domain of the isolated MBD variant may or may not comprise a substitution, as described above. It is generally understood that all MBD domain variants disclosed herein may additionally comprise flanking amino acid sequences, i.e. amino acid sequences that are located directly N- and/or C-terminal to the MBD core domain as defined herein. These sequences may correspond to the respective wildtype template sequences or may be different, heterologous or mutated sequences. In various embodiments, such polypeptides comprising the MBD core domain as defined herein are at least 50 amino acids in length, for example 60, 70, 80, 90, 100 or more amino acids. In addition to the MBD domain, these polypeptides may comprise further domains, such as those naturally occurring in the corresponding wildtype polypeptides.

The present invention relates to isolated MBD variants that have relative to the corresponding wildtype MBD an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC). In various embodiments, these variants are those described above having amino acid substitutions at the indicated positions, while in other embodiments they have alternative modifications.

Generally, in various embodiments, all isolated MBD variants disclosed herein may have an altered binding affinity, preferably increased affinity, for CpG dinucleotides and their complement in which

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC).

In various other embodiments, the isolated MBD variant has differential binding affinity for any two CpG dinucleotides and their complement selected from the following oxidized 5-methylated cytosine configurations:

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or
(l) both cytosine bases are 5-carboxylated (caC).

According to the present invention, the DNA molecule may be single-stranded or double-stranded, wherein double-stranded DNA is preferred. In a preferred embodiment, the DNA molecule is in its native state and is not chemically modified by any pretreatment.

Preferably, the cytosine nucleobases selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement are physiological cytosine states known in the art.

In various embodiments, the DNA molecule(s) can be labelled, preferably fluorescently labelled. In various embodiments, the labelled DNA molecules may have different oxi-mC configurations in a single CpG.

In various preferred embodiments, the isolated MBD variant comprises or consists of the amino acid sequence set forth in SEQ ID Nos. 46 to 52 and/or 53 to 55.

The invention further encompasses a conjugate comprising the isolated MBD variant as described above.

The term "conjugate" refers to a molecule that comprises the isolated MBD variant as described above coupled, typically covalently coupled, to another moiety, such as another protein, peptide, polypeptide, affinity ligand or detectable label or other moiety.

Such "moieties" are not particularly limited and can be easily chosen by a person skilled in the art in accordance with the particular application of interest. Further examples of such moieties include fluorescent dyes, fluorescent proteins such as e.g. green fluorescent protein (GFP), affinity ligands such as e.g. biotin, digoxigenin, dinitrophenol, magnetic particles, antibodies, including antibody fragments and antibody mimetics known in the art, protein tags, and proteins having enzymatic activities such as nuclease activities. Suitable protein tags in this respect include e.g. 18A-Tag, ACP-Tag, Avi-Tag, BCCP-Tag, Calmodulin-Tag (CaM-Tag), Chitin-binding-Protein-Tag (CBP-Tag), E-Tag, ELK16-Tag, ELP-Tag, FLAG-Tag, Flash-Tag, poly-glutamic acid-Tag, Glutathion-S-Transferase-Tag (GST-Tag), Green fluorescent protein-Tag (GFP-Tag), Hemagglutinin-Tag (HA-Tag), poly-Histidin-Tag (His-Tag), Isopeptag, Maltose binding protein-Tag (MBP-Tag), Myc-Tag, Nus-Tag, ProtA-Tag, ProtC-Tag, S-Tag, SBP-Tag, Snap-Tag, SpyTag, SofTag 1 and 3, Streptavidin-Tag (Strep-Tag), Strep-II-Tag, Tandem Affinity Purification-Tag (TAP-Tag), TC-Tag, Thioredoxin-Tag (TRX-Tag), Ty-Tag, V5-Tag, VSV-Tag, and Xpress-Tag, which are known in the art. Such "coupling moieties" can be coupled directly or indirectly to the MBD variant, or can be expressed as part of a conjugate comprising also the MBD variant. Methods for the coupling of respective "coupling moieties" and for the expression of respective conjugates are not particularly limited and are known in the art. For example, they include methods employing a His-Tag on the expressed conjugates and purification via NiNTA as known in the art, without being limited to this method.

In this context, MBD variants or conjugates can include non-natural amino acids with reactive groups. The coupling of the above compounds can be effected e.g. via click chemistry at said reactive groups. Suitable reactive groups in this context are not particularly limited and include e.g. azides, alkynes, alkenes, including strained alkynes and alkenes that have been genetically encoded in the form of non-natural amino acids.

Applications of respectively coupled MBD variants are not particularly limited and can be easily devised by the person skilled in the art in accordance with the respective application or scientific problem to be addressed.

In various embodiments, the conjugate further comprises an enzyme, preferably a nucleic acid modifying enzyme such as a nuclease (FokI nuclease, Micrococcal nuclease, DNaseI or other endo- and exonucleases), a methyltransferase (DNMT1, DNMT3a, DNMT3b, M.SssI, DamI or other Mtases known in the art), a deaminase, a biotin-ligase (BirA) or others. In another embodiment, the conjugate comprises a tag for later detection (e.g. SNAP tag, Sun tag, Flag tag, HA tag, His tag, etc.), or a fluorophore (e.g. GFP, mCherry, mClover, BFP, mRuby, DsRed etc.), which is bound to the isolated MBD variant.

Preferably, the isolated MBD variant or the conjugate as described above can be used for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

Accordingly, the invention also relates to the use of the isolated MBD variant as described above or the conjugate as described above for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

Further encompassed is a method for the determination of the methylation state of cytosine residues and/or oxidation state of the methyl group of 5-methylated cytosine residues in a CpG dinucleotide of interest and its complement in a DNA molecule, said method comprising

(a) providing a molecular probe comprising a Methyl-CpG binding domain (MBD) that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of said cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

Preferably, in the method described above, the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.
The binding affinity can be determined from a recombinantly expressed, isolated, and purified protein containing said MBD core domain using for example an electrophoretic mobility shift assay (EMSA) with fluorescently or radioactively labelled probes that contain a single CpG dinucleotide with one of the modification states specified above. Typically, the probe is added in nanomolar concentrations, e.g. 2 nM, and the MBD protein in micromolar to nanomolar concentrations, e.g. 1,024 nM or 128 nM (e.g. FIG. 6D) [7,8].
The selectivity of an immobilized MBD can also be determined using a mixture of said DNA probes of which at least one DNA probe is labelled with a different/distinguishable label such as a different fluorophore or other. Immobilization can be performed on cell surfaces, e.g. the bacterial cell surface, or on beads for purified proteins. In combination with fluorophore-labelled probes, a microfluidic or a flow cytometer enabled for fluorescence-activated cell sorting (FACS) can be used to quantify the selectivity of the MBD domain. If the detectable label is a unique barcode, e.g. a unique DNA sequence, the determination of binding selectivity can take place using a next-generation sequencing platform.
In various embodiments of the methods disclosed herein, the MBD variant and the DNA molecules are added in a molar ratio of MBD to DNA of between 2:1 and 500:1, more preferably of between 30:1 to 70:1, more preferably of between 40:1 to 60:1, most preferably of 50:1.
The invention further relates to a method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising

(a) providing a molecular probe comprising a Methyl-CpG binding domain (MBD) that binds to the region of the DNA molecule comprising said CpG dinucleotide of interest and differentially binds to different methylated cytosine and/or oxidized 5-methyl-cytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

In preferred embodiments, the binding affinity of the variant MBD to the DNA molecule comprising specific cytosine nucleobases as described above in a CpG dinucleotide differs from the binding affinity of the wildtype MBD to the DNA molecule comprising the same specific cytosine nucleobases in the CpG dinucleotide by at least 1%, preferably at least 5 %, more preferably at least 10 %, even more preferably at least 20 %, or even more preferably 50 % or more. The affinity may be expressed in terms of the dissociation constant Kd, as can for example be determined by various methods known in the art, including the aforementioned electromobility shift assays, fluorescence polarization assays, ELISA, footprinting, surface plasmon resonance based assays, microscale thermophoresis, calorimetric and other spectroscopic methods.
Preferably, the molecular probe of the method for the enrichment is immobilized on a substrate, more preferably on a functionalized capture bead. The molecular probe may thus comprise an affinity ligand that allows immobilization of the molecular probe on a substrate.

Affinity ligands for use according to these embodiments are not particularly limited and are known in the art. They include for example biotin, digoxigenin, dinitrophenol, magnetic particles, antibodies, including antibody fragments and antibody mimetics, and protein tags, e.g. as indicated above. Methods for the direct or indirect coupling of affinity ligands to MBD variants are not particularly limited and are known in the art. In this context, the term "coupled directly or indirectly" expressly includes the expression of conjugate molecules of MBD proteins and any particular proteinaceous affinity ligands. Further, the term "coupled indirectly" includes any coupling strategies employing linkers, binding via further affinity partners, and the like, which are known in the art.

In various embodiments, the enrichment step of the enrichment method comprises separating the complexes of the DNA molecules with the immobilized molecular probe from the non-complexed DNA molecules, the enrichment optionally including chromatography, centrifugation or magnetic bead separation.

In various other embodiments, the enrichment step may also comprise methods of DNA depletion. Particular methods include the use of His-tags as affinity ligand and depletion of DNAs bound to respective variant MBDs via Ni-NTA-beads, as well as the use of other protein tags as indicated above in connection with the respective affinity binding partners.

For example, DNA molecules in which specific (modified) cytosine nucleobases or specific oxidation states of the methyl group of 5-methylated cytosine residues in a CpG dinucleotide of interest and its complement are absent (or present) are depleted from a mixture containing DNA molecules in which the cytosine nucleobases are present, as well as DNA molecules in which the cytosine nucleobases are absent. In this manner, DNA molecules in which the cytosine nucleobases are present (or absent) are enriched in the mixture. Therefore, the MBD variant may be coupled directly or indirectly to a protein having nuclease activity, said protein allowing for the differential digestion of DNA molecules in which the cytosine nucleobases of interest are absent, wherein DNA molecules in which epigenetic modifications of said nucleobases of interest are present remain undigested.

Proteins having nuclease activity for use according to this embodiment are not particularly limited and are known in the art. They include for example restriction enzymes and cleavage domains of restriction enzymes, e.g. the cleavage domain of FokI. Methods for the direct or indirect coupling of proteins having nuclease activity to MBD variants are not particularly limited and are known in the art. In this context, the term "coupled directly or indirectly" expressly includes the expression of conjugate molecules of MBD variants and any particular proteins having nuclease activity. Further, the term "coupled indirectly" includes any coupling strategies employing linkers, binding via further affinity partners, and the like, which are known in the art. Moreover, methods for the digestion of DNA molecules in which specific modification (or a specific oxidation state) of said nucleobases is absent using said proteins having nuclease activity are not particularly limited and are known in the art. In this manner, DNA molecules in which the cytosine nucleobases of interest are present are enriched in the mixture.

The enriched DNA molecules, in which the cytosine nucleobases of interest are present, obtained by the method as described above, can be further used for any particular application of interest. For examples, said DNA molecules can be used for sequencing applications known in the art, including PCR and so-called Next Generation Sequencing methods, microarray analyses, or any methods for the analysis of nucleic acids known in the art.

The methods of the present invention can be used for the concurrent determination of the presence or absence of more than one particular cytosine nucleobase of interest in a given DNA molecule. In particular, several aliquots of a given DNA molecule can be provided, and the presence of different cytosine nucleobases of interest can be determined, respectively. As an example, the method of the present invention can be performed with respect to the detection of C, mC, hmC, fC, and caC, respectively, for a given DNA molecule.

The molecular probes used in the methods of the invention may generally comprise a variant MBD that comprises at least one amino acid substitution relative to the respective wildtype MBD, preferably an MBD variant as defined above. Preferably, the difference in binding affinity of the molecular probe is an increased binding affinity for a methylated state of cytosine and/or an oxidized state of 5-methylated cytosine, in particular 5-hydroxymethylcytosine, relative to the non-methylated cytosine and/or the non-oxidized 5-methylated cytosine.

Unless otherwise defined, all technical and scientific terms used herein have the same meaning as commonly understood by one of ordinary skill in the art. The singular terms "a", "an" and "the" include plural referents unless context clearly indicates otherwise. Similarly, the word "or" is intended to include "and" unless the context clearly indicates otherwise. The term "comprises" means "contains" or "includes". In case of conflict, the present specification, including explanations of terms, will control.

It is to be understood that the individual embodiments described herein can be combined with other embodiments of this application to form new embodiments, even if they were not disclosed contiguously. All embodiments and examples described for the MBD variant according to the invention also apply to the conjugate, use and/or method according to the invention, and vice versa. Additionally, it is to be understood that the various or preferred embodiments serve as examples and do not limit the scope of the invention.

The present invention will be further illustrated in the following non-limiting examples.

## EXAMPLES

Example 1

*Cloning of Expression and Display Vectors*

The MBD expression plasmids were derived from pET-21d(+) (Merck, Darmstadt, Germany), digested with *Xho*I and *Nco*I (New England Biolabs) to replace the T7 tag by Gibson assembly with a FLAG peptide-maltose-binding protein (MBP) tag, a factor Xa and a TEV recognition and cleavage site. The resulting vector pBeB1379 allowed expression of N-terminal MBP-MBD fusion proteins with a non-cleavable C-terminal 6xHis tag for purification.

The MBD surface display plasmid pBeB1383 was derived from a pBeB1379 digest with *Xho*I and *Nde*I to replace the FLAG-MBP tag, the factor Xa and the TEV recognition and cleavage site with an AIDA-I surface display cassette by Gibson assembly.

Both pBeB1379 and pBeB1383 contained a suitable multiple cloning site to incorporate, exchange, or isolate the coding sequences of said MBD variants.

*Expression of MBD proteins*

Similar to a protocol of [9], expression plasmids were transformed into *E. coli* BL21-Gold(DE3) (Agilent), and fresh overnight cultures of single clones were diluted to an optical density ($OD_{600}$) of 0.05 in 30 mL LB-Miller broth supplemented with 50 μg/mL carbenicillin, 1 mM $MgCl_2$ and 1 mM $ZnSO_4$. Cultures were grown at 37 °C (220 rpm) to an $OD_{600}$ of 0.5-0.6, briefly chilled on ice, and then induced by supplying 1 mM isopropyl β-d-1-thiogalactopyranoside (IPTG). Cultures were incubated at 25 °C (150 rpm) for at least 6 h or overnight, and cells were harvested and washed once by resuspension in 0.25 vol ice-cold 20 mM Tris-HCl (pH = 8.0). Pellets were resuspended in 2 mL binding buffer (20 mM Tris-HCl, 250 mM NaCl, 10% glycerol, adjusted to pH = 8.0, supplemented with 10 mM 2-mercaptoethanol, 5 mM imidazole and 0.1 % Triton X-100), and sonicated in a Bioruptor Pico (Diagenode) at 4 °C using 3 x 4 cycles (30 s pulse of 20-60 kHz, 25-200 W and 30 s rest). Suspensions were treated with 0.1 mg/mL lysozyme (Merck) and 10 U/mL DNase I (New England Biolabs) overnight. After centrifugation at 14,000 x g for 20 min at 4 °C, the cleared supernatants were retained, diluted with 1 vol binding buffer, mixed with 450 μL 50 % Ni-nitriloacetic acid (NTA) agarose resin (ThermoFisher), and incubated at 4 °C for 2 h. The resins were washed 2 x with 1 mL binding buffer containing 90 mM imidazole (20 min at 4 °C) and the fusion proteins were eluted in 2 x 0.2 mL and 1 x 0.4 mL binding buffer with 500 mM imidazole (10 min at 4 °C). Pure fractions (SDS PAGE) were combined and dialyzed against 3 x 15 mL 20 mM HEPES, 100 mM NaCl, 10% glycerol, adjusted to pH = 7.3, and 0.1% Triton X-100 in Slide-A-Lyzer MINI devices (3.5 kDa MWCO, ThermoFisher). An additional 1:2-1:5 dilution is recommended when scaling up this procedure to avoid precipitation during dialysis. The protein concentrations were determined with a BCA assay (ThermoFisher) and the proteins stocked at 15 μM after snap freezing in liquid nitrogen, at -80 °C (stable for several months).

*Electrophoretic Mobility Shift Assays*

24-mer oligodeoxynucleotide (ODNs) pairs containing one of the specific combinations of modified cytosine residues were combined at 1.5 μM of the labeled strand and 1.8 μM of the unlabeled strand in rudimentary EMSA buffer (20 mM HEPES, 30 mM KCl, 1 mM EDTA, 10 mM $(NH_4)_2SO_4$, pH = 7.3), incubated at 95 °C for 5 min, slowly brought to room temperature in a water bath for duplex formation, and subsequently diluted to 30 nM with respect to the labeled strand. The non-specific binding trap duplex was prepared by annealing a 24-mer poly(dA) with a 24-mer poly(dT) at equimolar ratios of 50 μM. EMSA were carried out according to a well-established protocol [9]. In brief, purified MBDs were diluted to 0, 2, 4, 8, 16, 32, 64, 128, 256, 512, and 1,024 nM in dialysis buffer with 0.1 mg/mL BSA (New England Biolabs) and incubated with 2 nM labeled duplex and 50 ng/μL poly(dA)·poly(dT) in EMSA buffer containing 1 mM dithiothreitol and 0.2 % Tween 20 in a final volume of 15 μL. The binding was allowed to equilibrate for 20 min at room temperature before 3 μL of a 6x loading dye (1.5 x TBE, pH = 7.5, 40% glycerol, 70 pg/mL bromophenol blue) were added on ice. These samples (10 μL) were loaded on pre-run 0.25 x TBE, 12% polyacrylamide gels and run at 240 V for 45 min at 4 °C in Mini-PROTEAN vertical electrophoresis cells (Bio-Rad). Gels were recorded on a Typhoon FLA-9500 laser scanner (GE Healthcare) equipped with a 473 nm laser and a 510 LP filter at 700-800 V PMT amplification without over-exposure. The fraction of bound duplex was determined using ImageQuant TL v8.1 1D Gel Analysis (GE Healthcare) applying rubber band background subtraction and manual peak detection with approximately equal peak areas across all lanes. The fraction of bound probe serves as a means to quantitate the relative affinity of a MBD at a fixed protein concentration (FIG. 6A). The removal of the N-terminal solubility tag such as MBP is optional and does not affect binding selectivity at the concentrations typically used in such assays.

*Surface Display Assay*

24-mer oligodeoxynucleotide (ODNs) pairs containing one of the specific combinations of modified cytosine residues and a 5'-biotin label were combined at equimolar ratios, annealed as described, and diluted to a final concentration of 400 nM in 6 x EMSA buffer and 300 ng/μL poly(dA)·poly(dT). Individual probes were labelled with fluorescent streptavidin (SAv) conjugates such as SAv-(R)-phycoerythrin (BioLegend) or SAv-AF488 (ThermoFisher) at 1.5-fold excess over the biotin component. Excess streptavidin was quenched after labeling at room temperature by addition of a 20-fold excess of free biotin (Car Roth). Surface display plasmids were transformed into *E. coli* BL21-Tuner(DE3) strains (Novagen) and fresh overnight cultures of single clones were diluted to an optical density ($OD_{600}$) of 0.05 in 3 mL LB-Miller broth supplemented with 50 μg/mL carbenicillin. Surface display was induced with 50 μM isopropyl β-d-1-thiogalactopyranoside (IPTG) at an OD600 of 0.5-0.7 for60 min at 30 °C. An amount of cells corresponding to an OD600 of 0.04 was harvested, washed and resuspended in 20 μL phosphate buffered saline (PBS) to which 10 μL of the staining mix was added which was prepared as described above according to the experimental demands. For example, this staining mix could contain 14 different ODNs labelled with SAv-AF488 and one ODN labeled with SAv-(*R*)-phycoerythrin at equimolar ratio. After 20 min at 22 °C, 700 rpm, the cells were washed again with PBS and analyzed on a flow cytometer (Sony Biotechnology) equipped with a 488 nm and 562 nm laser and suitable filter optics.

*Binding Assay Validation*

Both assays, the electrophoretic mobility shift assay (EMSA) and the surface display assay on FACS result in comparable estimates of the binding selectivity for a known binding MBD such as hMBD2 [2-81] containing SEQ ID NO:2 (FIG. 6B) as well as a known non-binding MBD such as hMBD3 [2-81] containing SEQ ID NO:3 (FIG. 6C) with the dynamic range of the EMSA at low protein concentrations, e.g. 128 nM (FIG. 6D) being most similar to the behavior observed under the conditions of said surface display assay (FIG. 6E).

*Identification of MBDs with Novel Binding Selectivity*

Immobilization on the bacterial cell surface was used to purify candidate binders from codon-degenerated MBD proteins containing SEQ ID Nos. 1, 2, 3, 4, or 5 using a modified procedure of the surface display assay described above. Codons corresponding to positions 12, 25, 26 (not SEQ ID NO:3), 27 (SEQ ID NO:3 only), and 37 were randomized using NNK-degenerated primers and standard molecular biology and protein engineering technologies. Individual clones harboring a single MBD variant of said libraries were sub-cloned in an expression plasmid such as pBeB1379, the protein product expressed and purified and subjected to EMSA (FIG. 7 and FIG. 8).

*Results*

- F105 is a MBD variant of SEQ ID NO:5 with selectivity for mC/caC, mC/mC, fC/caC and caC/caC.
- F106 is a MBD variant of SEQ ID NO:5 with selectivity for mC/hmC and mC/mC. It is the first example, in which an engineered MBD has higher affinity towards a CpG containing an oxidized mC species than for mC/mC (FIG. 9).
- F271 is a MBD variant of SEQ ID NO:5 with a single, high selectivity for mC/mC. This is a novel property within the domain of human MBDs containing SEQ ID Nos. 1, 2, 3, 4, or 5.
- F272 is a MBD variant with selectivity for a CpG containing one or two caC modifications in a single CpG. The most pronounced binding affinity is towards mC/caC.
- F273 is a MBD variant of SEQ ID NO:5 with selectivity for a CpG containing one or two caC modifications in a single CpG. The most pronounced binding affinity is towards caC/caC.
- F274 is a MBD variant of SEQ ID NO:5 with selectivity for mC/mC and mC/hmC within the domain of hemi-modified mC-containing CpGs as well as forhmC/hmC.
- F275 is a MBD variant of SEQ ID NO:2 with selectivity for mC/mC or a CpG containing one or two fC modifications in a single CpG.

Furthermore, within the set of codon-degenerated positions 12, 25, 26, 27, and 37, and MBD variants based on SEQ ID Nos. 2, 3, and 5 unpurified further variants with potentially high selectivity for mC/hmC, mC/fC, caC/caC and mC/caC were identified (FIG. 10).

The above results show that rationally identified positions within the MBD have the potential to alter the selectivity of various naturally occurring MBD protein domains for oxidized mC species in single CpGs. This enables both, the engineering of MBDs that are highly selective as compared to wildtype MBDs (e.g. F271) as well as MBDs that have a novel specificity with regards to the oxidation state of the cytosine bases in the CpG, i.e. MBDs with strongly reduced affinity for mC/mC, but increased affinity towards other configurations such as mC/hmC (e.g. F106), mC/caC (e.g. F272 or F273).

The amino acid sequences of the MBD core domain of variants F105, F106, F271, F272, F273, F274 and F275 are shown in FIG. 5.

Example 2

*Data analysis and Kd determinations*

All data was curated and analyzed with R v3.6.0. Given a single ligand binding site of the MBD protein domain and the absence of additional intramolecular interactions, the relationship between the total concentration of labeled duplex [L]o, added to the reaction, the total concentration of MBD [R]o, the fraction of bound ligand [RL]/[L]o at equilibrium and the dissociation constant Kd follows the quadratic equation [10]

$$[RL]^2 - [RL]\,([R]_0 + [L]_0 + K_d) + [R]_0[L]_0 = 0 \qquad \text{(Eq. 1)}$$

which has been used to determine the $K_d$ of MBD protein domains from fractional binding by Khrapunov *et al.* [11] as well as from free ligand, [L] = [L]o - [RL], by Yang *et al.* [12]. Here, we use the solution for the experimentally observed fraction of bound ligand, [RL]/[L]o, which is

$$[RL]/[L]_0 = ([R]_0 + [L]_0 + K_d - [\,([R]_0 + [L]_0 + K_d)^2 - 4[R]_0[L]_0]^{1/2}) \,/\, (2[L]_0) \qquad \text{(Eq. 2)}$$

to determine the $K_d$ by non-linear curve fitting using the Levenberg-Marquardt algorithm. We propagated the uncertainty associated with the $K_d$ estimates to the derived fold-changes according to the basic equation for error propagation [13].

*Results*

MBDs showed markedly different selectivity profiles for differentially modified CpGs despite their high degree of sequence conservation, particularly at residues interacting with the CpG. Therefore, it is of interest how RTT-associated single amino acid substitutions in MeCp2 would affect the selectivity profile of its MBD.

The mutants L124F, T158M, R133C and S134C [14] of human MeCp2[90-181] (wildtype sequence is set forth in SEQ ID NO:58 (FIG. 13)) were evaluated using EMSA and affinity measurements (Table 1 and FIG. 11). The single amino acid substitutions L124F (position 27 of SEQ ID NO:5), R133C (position 36 of SEQ ID NO:5) and S134C (position 37 of SEQ ID NO:5) are within the MBD core domain of hMeCp2 as defined herein (FIG. 12A). The single amino acid substitution T158M is present C-terminal to the MBD core domain as defined herein (position 61) (FIG. 12B).

Table 1: Dissociation constants $K_d$/nM

| **duplex** | **wildtype** | **R133C** | **S134C** |
|---|---|---|---|
| mC/mC | 27±4 | 118±12 | 117±9 |
| mC/hmC | 224±25 | 660±70 | 1,150±70 |
| mC/fC | 197±24 | 540±50 | 790±50 |
| hmC/hmC | 970±90 | 2,560±260 | 7,600±600 |
| hmC/fC | 540±60 | 1,630±90 | 4,600±400 |
| C/hmC | 1,080±100 | 8,100±500 | 7,600±700 |

**REFERENCES**

[1] M.J. Booth, M.R. Branco et al., Science 336(6083), 934-937 (2012).
[2] M. Yu, G.C. Hon et al., Cell 149(6), 1368-1380 (2012).
[3] M.J. Booth, G. Marsico et al., Nat Chem 6(5), 435-440 (2014).
[4] F. Neri, D. Incarnato et al., Cell Rep 10(5), 674-683 (2015).
[5] L. Shen, H. Wu et al., Cell 153(3), 692-706 (2013).
[6] H. Wu and Y. Zhang, Genes Dev. 25(23), 2436-2452 (2011).
[7] H. Hashimoto, J.E. Pais et al., Nature 506(7488), 391-395 (2014).
[8] V. Valinluck, H. Tsai et al., Nucl Acids Res 32(14), 4100-4108 (2004).
[9] A. Free et al., J Biol Chem 276, 3353-3360 (2001).
[10] E. C. Hulme, M.A. Trevethick, Br J Pharmacol 161, 1219-1237 (2010).
[11] S. Khrapunov et al., Biochemistry 53, 3379-3391 (2014).
[12] Y. Yang, T. G. Kucukkal et al., ACS Chem Biol 11, 2706-2715 (2016).
[13] P.R. Bevington, McGraw-Hill Education Ltd, (1969).
[14] J. Christodoulou, A. Grimm et al., Human mut 21, 466-472 (2003).

## Claims

1. Isolated Methyl-CpG binding domain (MBD) variant, comprising an MBD core domain that has at least 60 % sequence homology relative to any one of SEQ ID Nos.1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40, preferably 12, 19, 21, 22, 23, 25, 26, 27, 35, 36, 37, 38, and 39, more preferably 12, 25, 26, 27, 36 and 37, most preferably 12, 25, 26, 37, in SEQ ID NO:1.

2. The isolated MBD variant of claim 1, wherein said at least one amino acid substitution is selected from 12S, 12T, 12A, 12V, 12R, 25I, 25T, 25A, 25C, 25L, 26T, 26S, 26F, 26L, 27F, 36C, 37N, 37K, 37Q, 37R, 37V and 37C, preferably 12S, 12T, 12A, 12V, 12R, 25I, 25T, 25A, 25C, 25L, 26T, 26S, 26F, 26L, 37N, 37K, 37Q, 37R and 37V, more preferably 12S, 12T, 25I, 25T, 25A, 26T, 37N and 37K using the positional numbering of SEQ ID NO:1.

3. The isolated MBD variant of claim 1 or 2, wherein the MBD core domain has at least 65, 70, 75 or 80 % sequence homology or sequence identity to any one of SEQ ID Nos. 1-45.

4. The isolated MBD variant of any one of claims 1 to 3, wherein

   (1) the MBD core domain comprises any one or more of the amino acids 14R/K, 24D/E, 36R/K and 40E/Q/D using the positional numbering of SEQ ID NO:1; and/or
   (2) wherein the MBD core domain comprises any one or more of the amino acids 1P/K, 3L/V, 6G/D, 7W/F, 8R/Q/K/E/T, 9R/K, 17G, 27Y/F/L, 30P, 32G, 44Y/F and 45L/I using the positional numbering of SEQ ID NO:1; and/or
   (3) the MBD core domain comprises any one or more of the amino acids 1P/K, 2A/S/T, 3L,V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N, 30P, 31T/S/Q, 32G, 33D/K/L, 34R/K/A, 35I/F, 36R/K, 37S, 38K, 39V/P/S, 40E/Q/D/S, 41L, 42T/A/I, 43R/N/A, 44Y/F/V, 45L/I/F; and/or
   (4) said MBD variant has relative to the corresponding wildtype MBD an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

5. The isolated MBD variant of any one of claims 1 to 4, wherein

   (A) the variant has an altered binding affinity, preferably increased affinity, for CpG dinucleotides and their complement in which

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC); and/or

(B) the variant has differential binding affinity for any two CpG dinucleotides and their complement selected from the following oxidized 5-methylated cytosine configurations:

(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or
(l) both cytosine bases are 5-carboxylated (caC).

**6.** The isolated MBD variant of any one of claims 1 to 5, comprising or consisting of any one of the amino acid sequence set forth in SEQ ID Nos. 46 to 52 and/or 53 to 55.

**7.** Conjugate comprising the isolated MBD variant of any one of claims 1 to 6, wherein the conjugate optionally further comprises an enzyme, preferably a nuclease or methyltransferase, or a detectable label, preferably a fluorophore.

**8.** Use of the isolated MBD variant of any one of claims 1 to 6 or the conjugate of claim 7 for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

**9.** Method for the determination of the methylation state of cytosine residues and/or oxidation state of the methyl group of 5-methylated cytosine residues in a CpG dinucleotide of interest and its complement in a DNA molecule, said method comprising

(a) providing a molecular probe comprising a Methyl-CpG binding domain (MBD) that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of said cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

**10.** The method of claim 9, wherein the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.

**11.** Method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising

(a) providing a molecular probe comprising a Methyl-CpG binding domain (MBD) that binds to the region of the DNA molecule comprising said CpG dinucleotide of interest and differentially binds to different methylated cytosine and/or oxidized 5-methyl-cytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

**12.** The method of claim 11, wherein the molecular probe is immobilized on a substrate, wherein the molecular probe optionally comprises an affinity ligand that allows immobilization of the molecular probe on a substrate.

**13.** The method of any one of claims 11 to 12, wherein the enrichment step comprises separating the complexes of the DNA molecules with the immobilized molecular probe from the non-complexed DNA molecules, the enrichment optionally including chromatography, centrifugation or magnetic bead separation.

**14.** The method of any one of claims 9 to 13, wherein the molecular probe comprises a variant MBD that comprises at least one amino acid substitution relative to the respective wildtype MBD, preferably an MBD variant as defined in any one of claims 1 to 6.

**15.** The method of any one of claims 9 to 14, wherein the difference in binding affinity of the molecular probe is an increased binding affinity for a methylation state of a cytosine residue and/or an oxidized state of 5-methylated cytosine, preferably an oxidized state of 5-methylated cytosine, in particular 5-hydroxymethylcytosine, relative to the non-methylated cytosine and/or non-oxidized 5-methylated cytosine.

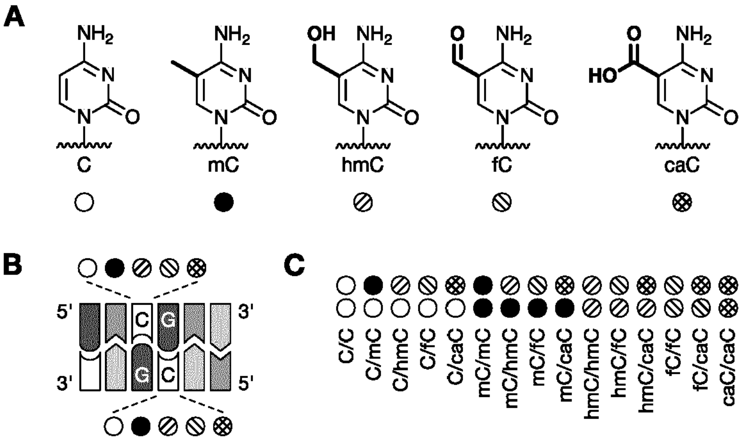


FIG. 1

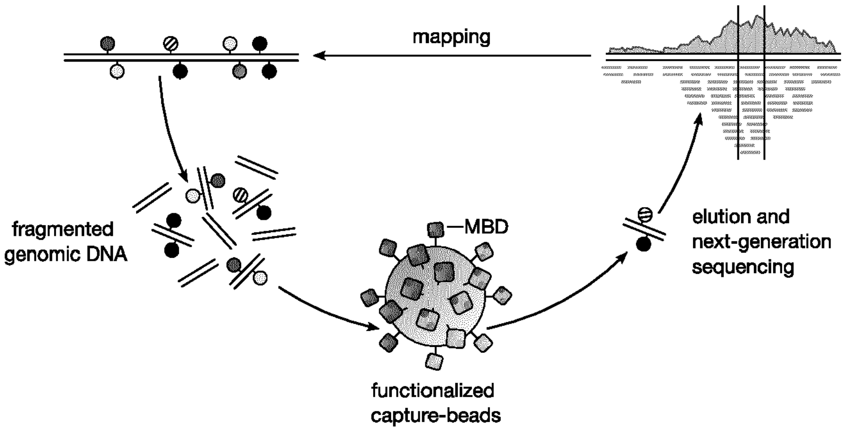


FIG. 2

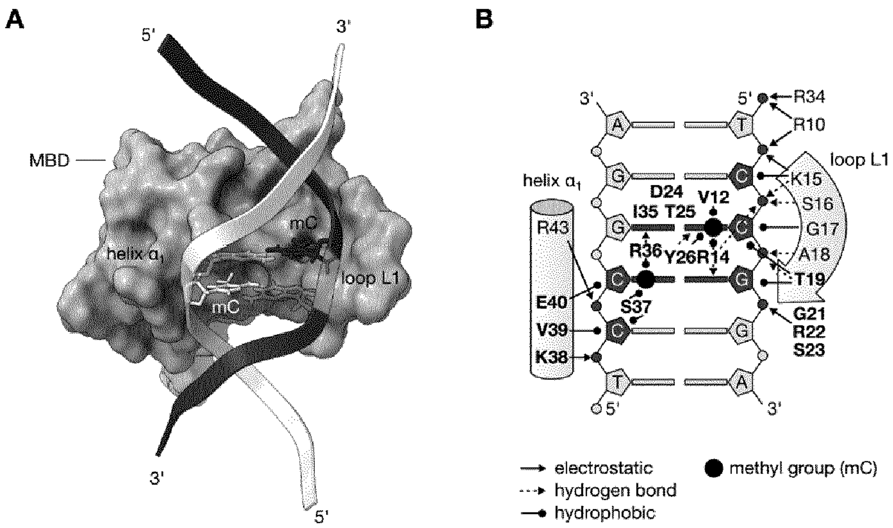


FIG. 3

FIG. 4

| SEQ ID NO. | MeCP2-MBD | species | sequence ref | structure ref | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 1 | hMBD1[2–81] | *H. sapiens* | CCDS59318.1 | PDB:1ig4 | P | A | L | G | P | G | W | K | R | R | E | V | F | R | K | S | G | A | T | C | G | R | S | D | T | Y | Y | Q | S | P | T | G | D | R | I | R | S | K | V | E | L | T | R | Y | L |
| 2 | hMBD2[146–225] | *H. sapiens* | CCDS11953.1 | PDB:6cnq | P | A | L | P | P | G | W | K | K | E | E | V | I | R | K | S | G | L | S | A | G | K | S | D | V | Y | Y | F | S | P | S | G | K | K | F | R | S | K | P | Q | L | A | R | Y | L |
| 3 | hMBD3[2–81] | *H. sapiens* | CCDS12072.1 | PDB:6ccg | P | A | L | P | Q | G | W | E | R | E | E | V | P | R | R | S | G | L | S | A | G | H | R | D | V | F | Y | Y | S | P | S | G | K | K | F | R | S | K | P | Q | L | A | R | Y | L |
| 4 | hMBD4[76–167] | *H. sapiens* | CCDS3058.1 | PDB:4lg7 | K | S | V | P | C | G | W | E | R | V | V | K | Q | R | L | F | G | K | T | A | G | R | F | D | V | Y | F | I | S | P | Q | G | L | K | F | R | S | K | S | S | L | A | N | Y | L |
| 5 | hMeCp2[90–181] | *H. sapiens* | CCDS14741.1 | PDB:3c2i | P | T | L | P | E | G | W | T | R | K | L | K | Q | R | K | S | G | R | S | A | G | K | Y | D | V | Y | L | I | N | P | Q | G | K | A | F | R | S | K | V | E | L | I | A | Y | F |
| 6 | mMbd1[1–92] | *M. musculus* | CCDS50321.1 | | P | A | L | G | P | G | W | K | R | R | E | S | F | R | K | S | G | A | S | F | G | R | S | D | I | Y | Y | Q | S | P | T | G | E | K | I | R | S | K | V | E | L | T | R | Y | L |
| 7 | mMbd2[148–240] | *M. musculus* | CCDS29335.1 | | P | A | L | P | P | G | W | K | K | E | E | V | I | R | K | S | G | L | S | A | G | K | S | D | V | Y | Y | F | S | P | S | G | K | K | F | R | S | K | P | Q | L | A | R | Y | L |
| 8 | mMbd3[1–92] | *M. musculus* | CCDS24020.1 | | P | A | L | P | Q | G | W | E | R | E | E | V | P | R | R | S | G | L | S | A | G | H | R | D | V | F | Y | Y | S | P | S | G | K | K | F | R | S | K | P | Q | L | A | R | Y | L |
| 9 | mMbd4[112–194] | *M. musculus* | CCDS39603.1 | PDB:3vxx | K | P | V | P | C | G | W | E | R | V | V | K | Q | R | L | S | G | K | T | A | G | K | F | D | V | Y | F | I | S | P | Q | G | L | K | F | R | S | K | R | S | L | A | N | Y | L |
| 10 | mMecp2[89–183] | *M. musculus* | CCDS41017.1 | | P | T | L | P | E | G | W | T | R | K | L | K | Q | R | K | S | G | R | S | A | G | K | Y | D | V | Y | L | I | N | P | Q | G | K | A | F | R | S | K | V | E | L | I | A | Y | F |
| 11 | Q66HB8_RAT | *R. norvegicus* | | | P | A | L | G | P | G | W | K | R | R | E | A | F | R | K | S | G | A | S | C | G | R | S | D | I | Y | Y | R | S | P | T | G | E | K | I | R | S | K | I | E | L | T | R | Y | L |
| 12 | D4A986_RAT | *R. norvegicus* | | | P | A | L | P | P | G | W | K | K | E | E | V | I | R | K | S | G | L | S | A | G | K | S | D | V | Y | Y | F | S | P | S | G | K | K | F | R | S | K | P | Q | L | A | R | Y | L |
| 13 | UPI00001830DE | *R. norvegicus* | | | P | A | L | P | Q | G | W | E | R | E | E | V | P | R | R | S | G | L | S | A | G | H | R | D | V | F | Y | Y | S | P | S | G | K | K | F | R | S | K | P | Q | L | A | R | Y | L |
| 14 | H2QNC3_PANTR | *P. troglodytes* | | | K | S | V | P | C | G | W | E | R | V | V | K | Q | R | L | F | G | K | T | A | G | R | F | D | V | Y | F | I | S | P | Q | G | L | K | F | R | S | K | S | S | L | A | N | Y | L |
| 15 | K7CR55_PANTR | *P. troglodytes* | | | P | A | L | G | P | G | W | K | R | R | E | V | F | R | K | S | G | A | T | C | G | R | S | D | T | Y | Y | Q | S | P | T | G | D | R | I | R | S | K | V | E | L | T | R | Y | L |
| 16 | A0A2I3SLX7_PANTR | *P. troglodytes* | | | P | T | L | P | E | G | W | T | R | K | L | K | Q | R | K | S | G | R | S | A | G | K | Y | D | V | Y | L | I | N | P | Q | G | K | A | F | R | S | K | V | E | L | I | A | Y | F |
| 17 | A0A2J8INA6_PANTR | *P. troglodytes* | | | P | T | L | P | E | G | W | T | R | K | L | K | Q | R | K | S | G | R | S | A | G | K | Y | D | V | Y | L | I | N | P | Q | G | K | A | F | R | S | K | V | E | L | I | A | Y | F |
| 18 | Q7T2T7_DANRE | *D. rerio* | | | P | S | L | P | Q | G | W | T | R | K | L | K | Q | R | K | S | G | R | S | A | G | K | F | D | V | Y | L | I | N | P | E | G | K | A | F | R | S | K | V | E | L | M | A | Y | F |
| 19 | Q6TGX7_DANRE | *D. rerio* | | | S | A | L | P | N | G | W | K | M | E | E | V | T | R | K | S | G | L | S | A | G | K | S | D | V | Y | Y | F | S | P | T | G | K | K | F | R | S | K | P | Q | L | V | R | Y | L |
| 20 | H2SUB2_TAKRU | *T. rubripes* | | | T | A | L | P | K | G | W | K | M | E | E | V | T | R | K | S | G | L | S | A | G | K | S | D | V | Y | Y | F | S | P | S | G | K | K | F | R | S | K | P | Q | L | A | R | Y | L |
| 21 | Q9YGC6_XENLA | *X. laevis* | | | P | T | L | P | E | G | W | T | R | K | L | K | Q | R | K | S | G | R | S | A | G | K | F | D | V | Y | L | I | N | P | N | G | K | A | F | R | S | K | V | E | L | I | A | Y | F |
| 22 | Q8AYP2_XENLA | *X. laevis* | | | S | A | L | P | Q | G | W | K | K | E | E | V | T | R | R | S | G | L | S | A | G | K | S | D | V | Y | Y | Y | S | P | S | G | K | K | F | R | S | K | P | Q | L | A | R | Y | L |
| 23 | Q8AYT1_XENLA | *X. laevis* | | | P | A | L | P | A | G | W | K | K | E | E | V | I | R | K | S | G | L | S | A | G | K | S | D | V | Y | Y | Y | S | P | N | G | K | K | F | R | S | K | P | Q | L | A | R | Y | L |
| 24 | Q7ZYD4_XENLA | *X. laevis* | | | P | L | L | G | P | G | W | K | R | R | N | V | V | R | K | S | G | A | T | C | G | H | S | D | T | Y | Y | R | S | P | A | G | K | K | I | R | S | R | I | E | L | A | K | Y | L |
| 25 | UPI00084D2E70 | *X. laevis* | | | P | T | L | P | E | G | W | T | R | K | L | K | Q | R | K | S | G | R | S | A | G | K | Y | D | V | Y | L | I | H | P | N | G | K | A | F | R | S | K | V | E | L | I | A | Y | F |
| 26 | Q5EFL0_CHICK | *G. gallus* | | PDB:2ky8 | P | A | L | P | P | G | W | K | K | E | E | V | I | R | K | S | G | L | S | A | G | K | S | D | V | Y | Y | F | S | P | S | G | K | K | F | R | S | K | P | Q | L | A | R | Y | L |
| 27 | UPI0005EE466F | *S. purpuratus* | | | P | G | L | P | A | G | W | K | R | E | E | V | I | R | K | S | G | L | S | A | G | K | T | D | V | Y | Y | Y | S | P | C | G | K | K | L | R | S | K | P | Q | L | A | R | F | I |
| 28 | W4YH51_STRPU | *S. purpuratus* | | | W | P | L | A | N | G | W | R | R | Q | T | I | I | R | Q | L | G | P | G | DR | I | K | G | D | V | I | Y | Y | A | P | C | G | K | K | L | R | T | Y | P | E | V | V | R | Y | I |
| 29 | F5HM38_ANOGA | *A. gambiae* | | | A | A | L | P | K | G | W | Q | R | E | E | V | L | R | K | T | G | L | S | A | G | K | V | D | V | Y | Y | Y | S | P | T | G | K | K | I | E | S | K | P | Q | L | A | R | A | L |
| 30 | Q23590_CAEEL | *C. elegans* | | | L | P | L | Q | L | G | W | R | R | Q | T | C | V | R | S | I | A | S | A | G | V | K | G | D | V | S | Y | F | A | P | C | G | K | K | L | S | T | Y | S | E | V | V | R | Y | L |
| 31 | MBD4_ARATH | *A. thaliana* | | | P | K | T | P | K | G | F | K | R | S | L | V | L | R | K | D | Y | S | - | - | - | K | M | D | T | Y | Y | F | T | P | T | G | K | K | L | R | S | R | N | E | I | A | A | F | V |
| 32 | MBD1_ARATH | *A. thaliana* | | | P | R | T | P | R | G | F | K | R | S | L | I | L | R | K | D | Y | S | - | - | - | K | M | D | A | Y | Y | I | T | P | T | G | K | K | L | K | S | R | N | E | I | A | A | F | I |

| SEQ ID NO. | HAT-MBD group | species | sequence ref | structure ref | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 33 | hBAZ2A[549–624] | *H. sapiens* | CCDS44924.1 | PDB:5agq | L | P | L | Q | H | G | W | R | R | E | V | R | I | K | K | G | S | - | H | R | W | Q | G | E | T | W | Y | Y | G | P | C | G | K | R | M | K | Q | F | P | E | V | I | K | Y | L |
| 34 | hBAZ2B[742–817] | *H. sapiens* | CCDS2209.2 | | I | P | L | E | Y | G | W | Q | R | E | T | R | I | R | N | F | G | - | G | R | L | Q | G | E | V | A | Y | Y | A | P | C | G | K | K | L | R | Q | Y | P | E | V | I | K | Y | L |
| 35 | mBaz2a[539–614] | *H. sapiens* | CCDS36088.1 | | L | P | L | Q | H | G | W | R | R | E | V | R | I | K | K | G | S | - | H | R | W | Q | G | E | T | W | Y | Y | G | P | C | G | K | R | M | K | Q | F | P | E | V | I | K | Y | L |
| 36 | A0A1D5NW81_CHICK | *G. gallus* | | | F | P | L | Q | H | G | W | R | R | E | V | R | I | K | R | G | N | - | H | R | W | Q | G | E | T | W | Y | Y | G | P | C | G | K | R | M | K | Q | F | P | E | V | I | K | Y | L |
| 37 | UPI00015526AD | *R. norvegicus* | | | L | P | L | Q | H | G | W | R | R | E | V | R | I | K | K | G | S | - | H | R | W | Q | G | E | T | W | Y | Y | G | P | C | G | K | R | M | K | Q | F | P | E | V | I | K | Y | L |

| SEQ ID NO. | HMT-MBD group | species | sequence ref | structure ref | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 38 | hSETDB1[597–672] | *H. sapiens* | CCDS972.1 | | V | P | L | L | Y | D | F | R | R | M | T | A | R | R | R | V | N | - | R | K | M | G | F | H | V | I | Y | K | T | P | C | G | L | C | L | R | T | M | Q | E | I | E | R | Y | L |
| 39 | hSETDB2[163–228] | *H. sapiens* | CCDS9417.1 | | L | P | I | K | C | H | F | Q | R | R | H | A | K | T | N | S | H | - | - | S | S | A | L | H | V | S | Y | K | T | P | C | G | R | S | L | R | N | V | E | E | V | F | R | Y | L |
| 40 | mSetdb1[615–690] | *H. sapiens* | CCDS17613.1 | | V | P | L | L | Y | D | F | R | R | M | T | A | R | R | R | V | N | - | R | K | M | G | F | H | V | I | Y | K | T | P | C | G | L | C | L | R | T | M | Q | E | I | E | R | Y | L |
| 41 | mSetdb2[148–220] | *H. sapiens* | CCDS84142.1 | | L | P | I | R | C | H | F | Q | R | R | H | A | K | T | N | S | H | - | - | S | S | A | L | H | V | N | Y | K | T | P | C | G | R | N | L | R | N | M | E | E | V | F | H | Y | L |
| 42 | H2UKE2_TAKRU | *T. rubripes* | | | T | P | L | L | Y | D | F | R | R | M | T | G | R | R | K | V | N | - | R | K | M | S | F | H | V | I | Y | K | A | P | C | G | L | C | L | R | N | M | S | E | I | Q | H | Y | L |
| 43 | SETB1_XENLA | *X. laevis* | | | V | P | L | L | Y | D | F | R | R | M | T | A | R | R | R | V | N | - | R | K | M | G | F | H | V | I | Y | K | S | P | C | G | L | S | L | R | T | M | P | E | I | E | R | Y | L |
| 44 | H9L3I9_CHICK | *G. gallus* | | | I | P | L | L | Y | D | F | R | R | M | T | A | R | R | R | V | N | - | R | K | M | G | F | H | V | I | Y | K | T | P | C | G | L | C | L | R | S | M | A | E | I | E | R | Y | L |
| 45 | SETDB1_RAT | *R. norvegicus* | | | V | P | L | L | Y | D | F | R | R | M | T | A | R | R | R | V | N | - | R | K | M | G | F | H | V | I | Y | K | T | P | C | G | L | C | L | R | T | M | Q | E | I | E | R | Y | L |

| SEQ ID NO. | MBD variant | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 46 | F105 | P | T | L | P | E | G | W | T | R | K | L | **T** | Q | R | K | S | G | R | S | A | G | K | Y | D | **T** | **T** | L | I | N | P | Q | G | K | A | F | R | **K** | K | V | E | L | I | A | Y | F |
| 47 | F106 | P | T | L | P | E | G | W | T | R | K | L | **T** | Q | R | K | S | G | R | S | A | G | K | Y | D | **A** | Y | L | I | N | P | Q | G | K | A | F | R | **N** | K | V | E | L | I | A | Y | F |
| 48 | F271 | P | T | L | P | E | G | W | T | R | K | L | K | Q | R | K | S | G | R | S | A | G | K | Y | D | **C** | **S** | L | I | N | P | Q | G | K | A | F | R | **Q** | K | V | E | L | I | A | Y | F |
| 49 | F272 | P | T | L | P | E | G | W | T | R | K | L | **A** | Q | R | K | S | G | R | S | A | G | K | Y | D | **C** | **F** | L | I | N | P | Q | G | K | A | F | R | **R** | K | V | E | L | I | A | Y | F |
| 50 | F273 | P | T | L | P | E | G | W | T | R | K | L | K | Q | R | K | S | G | R | S | A | G | K | Y | D | **L** | **T** | L | I | N | P | Q | G | K | A | F | R | **R** | K | V | E | L | I | A | Y | F |
| 51 | F274 | P | T | L | P | E | G | W | T | R | K | L | **V** | Q | R | K | S | G | R | S | A | G | K | Y | D | V | **L** | L | I | N | P | Q | G | K | A | F | R | **R** | K | V | E | L | I | A | Y | F |
| 52 | F275 | P | A | L | P | P | G | W | K | K | E | E | **R** | I | R | K | S | G | L | S | A | G | K | S | D | V | Y | Y | F | S | P | S | G | K | K | F | R | **V** | K | P | Q | L | A | R | Y | L |

FIG. 5

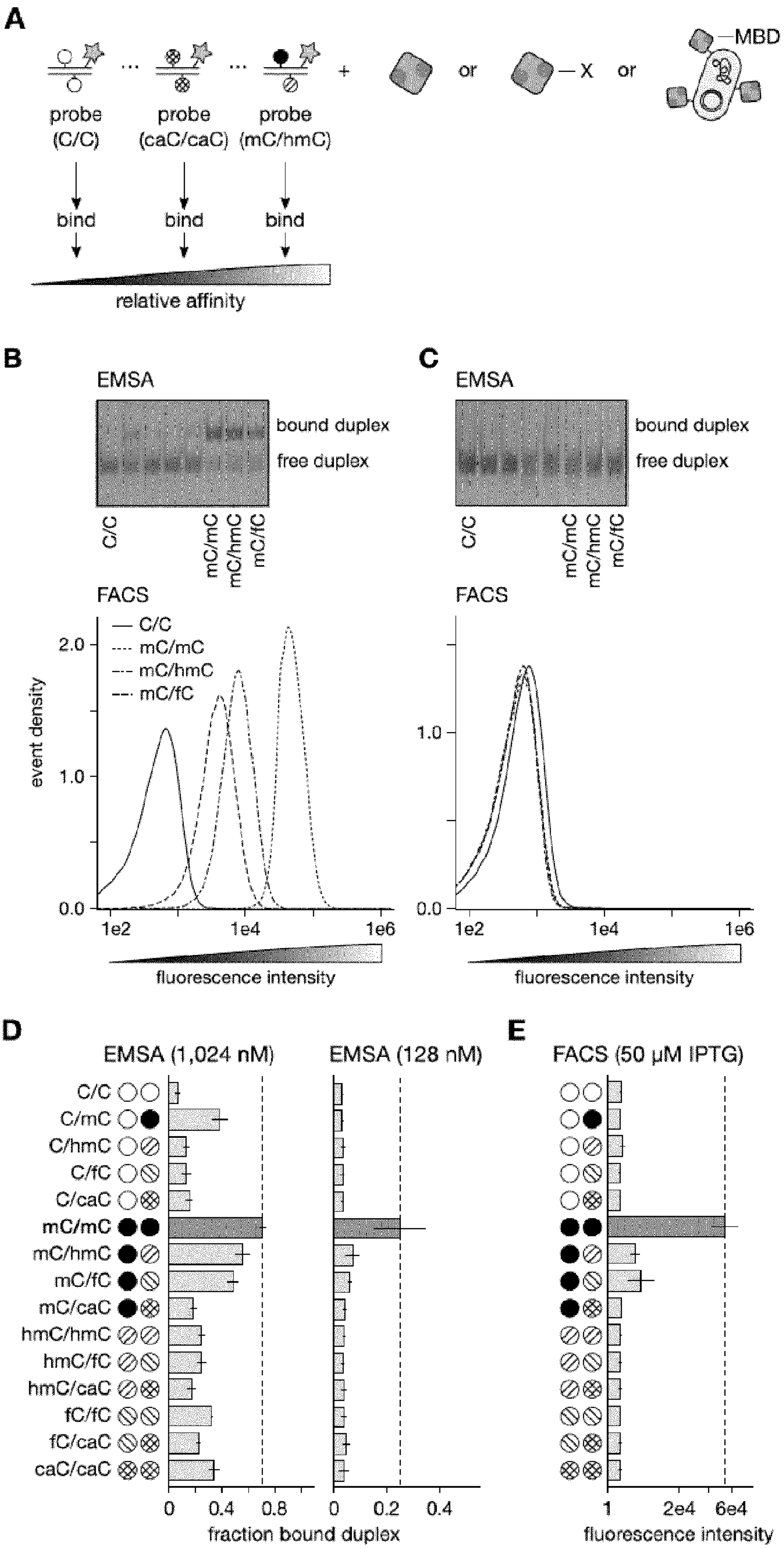
A
probe
(C/C)
probe
(caC/caC)
probe
(mC/hmC)
bind
bind
bind
relative affinity
or
—X
or
—MBD
B
EMSA
bound duplex
free duplex
C/C
mC/mC
mC/hmC
mC/fC
FACS
C/C
mC/mC
mC/hmC
mC/fC
event density
fluorescence intensity
C
EMSA
bound duplex
free duplex
FACS
D
EMSA (1,024 nM)
EMSA (128 nM)
C/C
C/mC
C/hmC
C/fC
C/caC
mC/mC
mC/hmC
mC/fC
mC/caC
hmC/hmC
hmC/fC
hmC/caC
fC/fC
fC/caC
caC/caC
fraction bound duplex
E
FACS (50 µM IPTG)
fluorescence intensity

FIG. 6

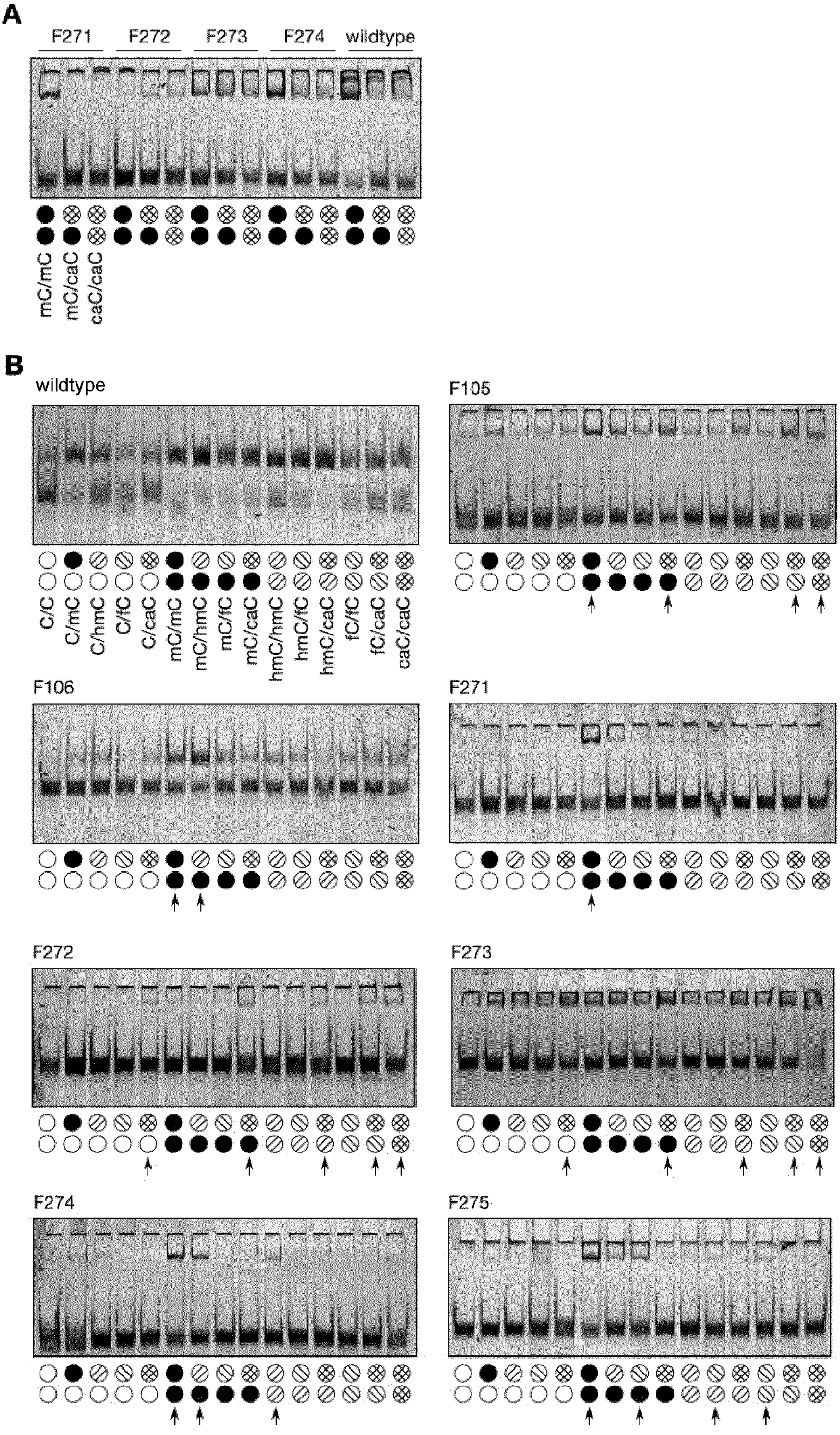
A
F271
F272
F273
F274
wildtype
mC/mC
mC/caC
caC/caC
B
wildtype
F105
C/C
C/mC
C/hmC
C/fC
C/caC
mC/mC
mC/hmC
mC/fC
mC/caC
hmC/hmC
hmC/fC
hmC/caC
fC/fC
fC/caC
caC/caC
F106
F271
F272
F273
F274
F275

FIG. 7

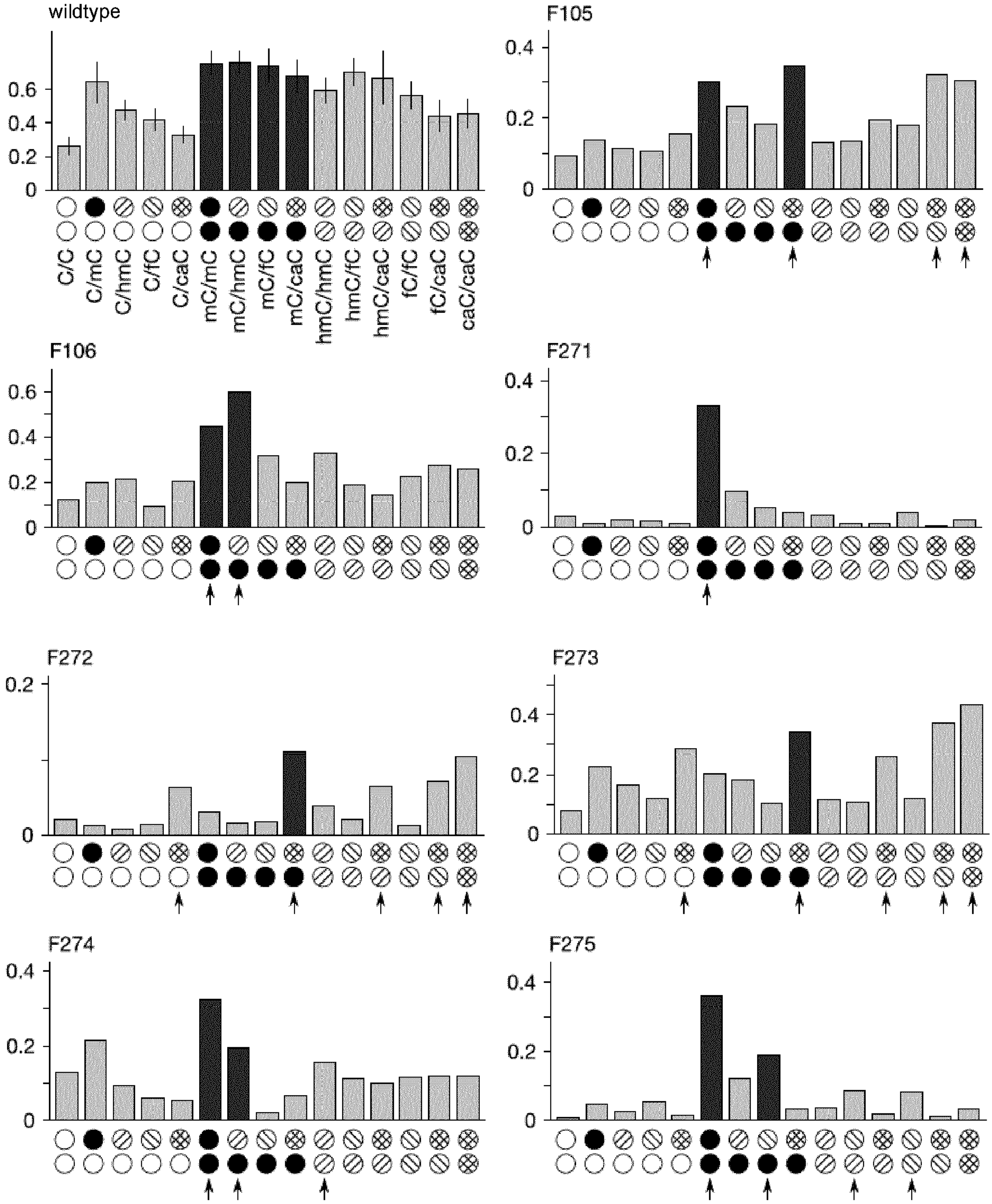
wildtype
0.6
0.4
0.2
0
C/C
C/mC
C/hmC
C/fC
C/caC
mC/mC
mC/hmC
mC/fC
mC/caC
hmC/hmC
hmC/fC
hmC/caC
fC/fC
fC/caC
caC/caC
F105
0.4
0.2
0
F106
0.6
0.4
0.2
0
F271
0.4
0.2
0
F272
0.2
0
F273
0.4
0.2
0
F274
0.4
0.2
0
F275
0.4
0.2
0

FIG. 8

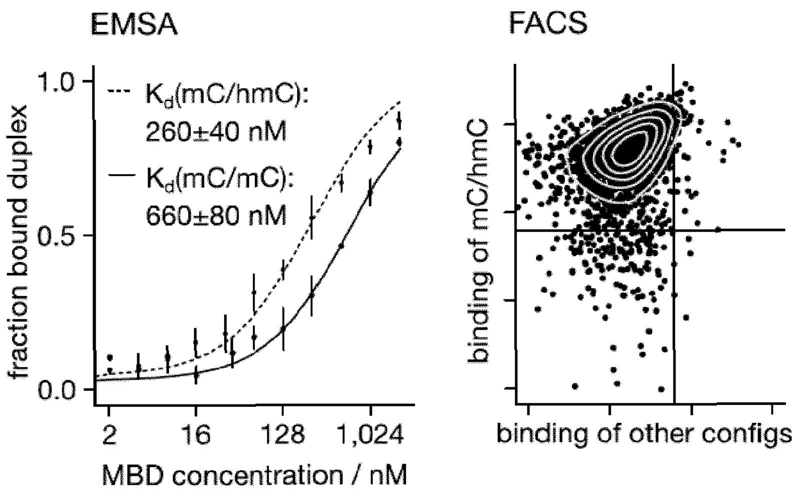
EMSA
FACS
1.0
0.5
0.0
fraction bound duplex
--- $K_d$(mC/hmC): 260±40 nM
— $K_d$(mC/mC): 660±80 nM
2
16
128
1,024
MBD concentration / nM
binding of mC/hmC
binding of other configs

FIG. 9

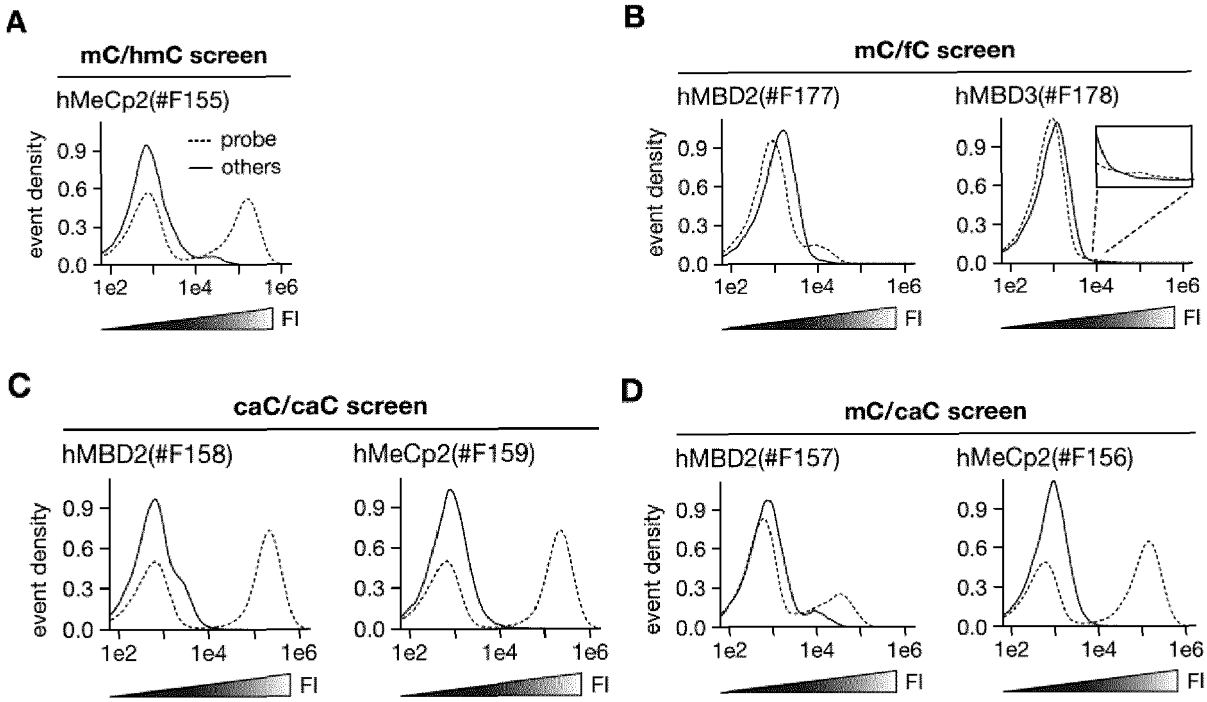
A
mC/hmC screen
hMeCp2(#F155)
---- probe
— others
event density
0.9
0.6
0.3
0.0
1e2
1e4
1e6
FI
B
mC/fC screen
hMBD2(#F177)
hMBD3(#F178)
C
caC/caC screen
hMBD2(#F158)
hMeCp2(#F159)
D
mC/caC screen
hMBD2(#F157)
hMeCp2(#F156)

FIG. 10

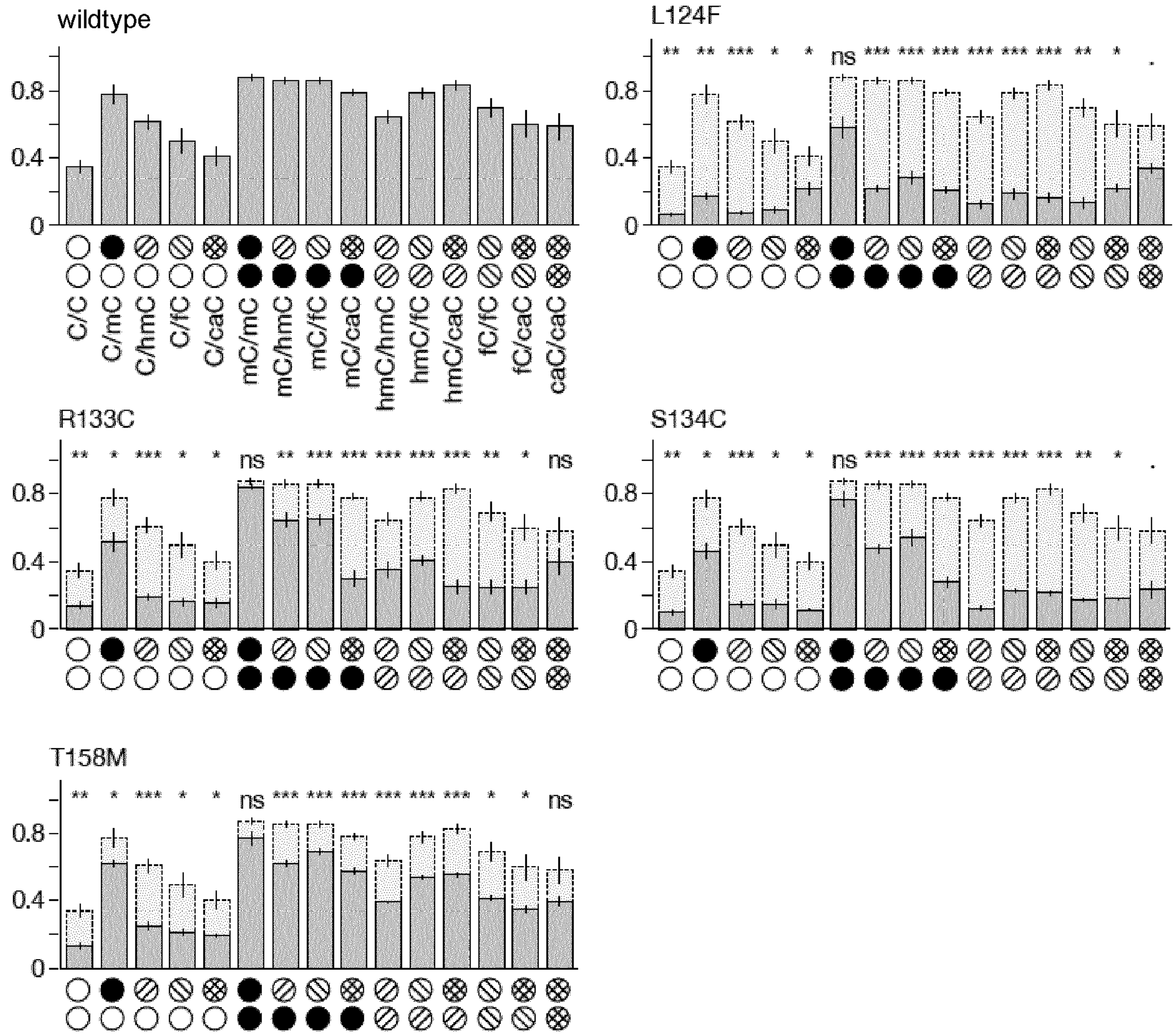
wildtype
L124F
R133C
S134C
T158M
0
0.4
0.8
C/C
C/mC
C/hmC
C/fC
C/caC
mC/mC
mC/hmC
mC/fC
mC/caC
hmC/hmC
hmC/fC
hmC/caC
fC/fC
fC/caC
caC/caC
ns

FIG. 11

FIG. 12

**A**

| SEQ ID NO. | MBD variant | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 53 | L124F | P | T | L | P | E | G | W | T | R | K | L | K | Q | R | K | S | G | R | S | A | G | K | Y | D | V | Y | **F** | I | N | P | Q | G | K | A | F | R | S | K | V | E | L | I | A | Y | F |
| 54 | R133C | P | T | L | P | E | G | W | T | R | K | L | K | Q | R | K | S | G | R | S | A | G | K | Y | D | V | Y | L | I | N | P | Q | G | K | A | F | **C** | S | K | V | E | L | I | A | Y | F |
| 55 | S134C | P | T | L | P | E | G | W | T | R | K | L | K | Q | R | K | S | G | R | S | A | G | K | Y | D | V | Y | L | I | N | P | Q | G | K | A | F | R | **C** | K | V | E | L | I | A | Y | F |

**B**

| SEQ ID NO. | MBD variant | |
|---|---|---|
| 56 | T158M | D R G P M Y D D P T L P E G W T R K L K Q R K S G R S A G K Y D V Y L I N P Q G K A F R S K V E L I A Y<br>F E K V G D T S L D P N D F D F **M** V T G R G S P S R R E Q K P P K K P K S P K A |

FIG. 13

| SEQ ID NO. | MeCP2-MBD | |
|---|---|---|
| 57 | hMBD2[146–225] | S G K R M D C P A L P P G W K K E E V I R K S G L S A G K S D V Y Y F S P S G K K F R S K P Q L A R Y L<br>G N T V D L S S F D F R T G K M M P S K L Q K N K Q R L |
| 58 | hMeCp2[90–181] | D R G P M Y D D P T L P E G W T R K L K Q R K S G R S A G K Y D V Y L I N P Q G K A F R S K V E L I A Y<br>F E K V G D T S L D P N D F D F T V T G R G S P S R R E Q K P P K K P K S P K A |
| 59 | hMBD1[2–81] | A E D W L D C P A L G P G W K R R E V F R K S G A T C G R S D T Y Y Q S P T G D R I R S K V E L T R Y L<br>G P A C D L T L F D F K Q G I L C Y P A P K A H P V A V |
| 60 | hMBD3[2–81] | E R K R W E C P A L P Q G W E R E E V P R R S G L S A G H R D V F Y Y S P S G K K F R S K P Q L A R Y L<br>G G S M D L S T F D F R T G K M L M S K M N K S R Q R V |
| 61 | hMBD4[76–167] | A T A G T E C R K S V P C G W E R V V K Q R L F G K T A G R F D V Y F I S P Q G L K F R S K S S L A N Y<br>L H K N G E T S L K P E D F D F T V L S K R G I K S R Y K D C S M A A L T S H L |

Europäisches Patentamt
European Patent Office
Office européen des brevets

# EUROPEAN SEARCH REPORT

Application Number
EP 19 22 0082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>A | WO 2016/210124 A2 (MASSACHUSETTS INST TECHNOLOGY [US])<br>29 December 2016 (2016-12-29)<br>* the whole document *<br>----- | 1-5,7,8<br><br><br>6 | INV.<br>C07K14/47<br>G01N33/53 |
| X | T. C. GALVAO ET AL: "Structure-specific binding of MeCP2 to four-way junction DNA through its methyl CpG-binding domain",<br>NUCLEIC ACIDS RESEARCH,<br>vol. 33, no. 20,<br>27 November 2005 (2005-11-27), pages 6603-6609, XP055704860,<br>ISSN: 0305-1048, DOI: 10.1093/nar/gki971<br>* the whole document *<br>----- | 1-7 | |
| X | NAOYUKI FUJITA ET AL: "Mechanism of Transcriptional Regulation by Methyl-CpG Binding Protein MBD1",<br>MOLECULAR AND CELLULAR BIOLOGY,<br>vol. 20, no. 14,<br>1 January 2000 (2000-01-01), pages 5107-5118, XP055704784,<br>* the whole document *<br>----- | 1-5,7 | TECHNICAL FIELDS SEARCHED (IPC)<br>C07K |
| X | S KUDO: "Heterogeneity in residual function of MeCP2 carrying missense mutations in the methyl CpG binding domain",<br>JOURNAL OF MEDICAL GENETICS,<br>vol. 40, no. 7, 1 July 2003 (2003-07-01), pages 487-493, XP055705067,<br>DOI: 10.1136/jmg.40.7.487<br>* the whole document *<br>----- | 1-6 | |

1 ~~The present search report has been drawn up for all claims~~

| Place of search | Date of completion of the search | Examiner |
|---|---|---|
| The Hague | 15 June 2020 | Wiame, Ilse |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 22 0082

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-8

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 19 22 0082

The Search Division considers that the present European patent application does not comply with the requirements of unity of invention and relates to several inventions or groups of inventions, namely:

1. claims: 1-8

Isolated Methyl-CpG binding domain (MBD) variant, comprising an MBD core domain that has at least 60% sequence homology relative to any one of SEQ ID Nos.1-45 and comprising at least one amino acid substitution relative to the corresponding wildtype MBD in at least one of the positions corresponding to positions 12, 14, 19, 21, 22, 23, 24, 25, 26, 27, 35, 36, 37, 38, 39 and 40 in SEQ ID NO:1.

---

2. claims: 9, 10(completely); 14, 15(partially)

Method for the determination of the methylation state of cytosine residues and/or oxidation state of the methyl group of 5-methylated cytosine residues in a CpG dinucleotide of interest and its complement in a DNA molecule, said method comprising
(a) providing a molecular probe comprising a Methyl-CpG binding domain (MBD) that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of said cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

---

3. claims: 11-13(completely); 14, 15(partially)

Method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising
(a) providing a molecular probe comprising a Methyl-CpG binding domain (MBD) that binds to the region of the DNA molecule comprising said CpG dinucleotide of interest and differentially binds to different methylated cytosine and/or oxidized 5-methyl-cytosine configurations in the CpG

Europäisches Patentamt
European Patent Office
Office européen des brevets

# LACK OF UNITY OF INVENTION SHEET B

Application Number
EP 19 22 0082

The Search Division considers that the present European patent application does not comply with the requirements of unity of invention and relates to several inventions or groups of inventions, namely:

dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

---

# ANNEX TO THE EUROPEAN SEARCH REPORT ON EUROPEAN PATENT APPLICATION NO. EP 19 22 0082

This annex lists the patent family members relating to the patent documents cited in the above-mentioned European search report. The members are as contained in the European Patent Office EDP file on
The European Patent Office is in no way liable for these particulars which are merely given for the purpose of information.

15-06-2020

| Patent document cited in search report | Publication date | Patent family member(s) | Publication date |
|---|---|---|---|
| WO 2016210124 A2 | 29-12-2016 | US 2017030898 A1<br>WO 2016210124 A2 | 02-02-2017<br>29-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **M.J. BOOTH ; M.R. BRANCO et al.** *Science,* 2012, vol. 336 (6083), 934-937
- **M. YU ; G.C. HON et al.** *Cell,* 2012, vol. 149 (6), 1368-1380
- **M.J. BOOTH ; G. MARSICO et al.** *Nat Chem,* 2014, vol. 6 (5), 435-440
- **F. NERI ; D. INCARNATO et al.** *Cell Rep,* 2015, vol. 10 (5), 674-683
- **L. SHEN ; H. WU et al.** *Cell,* 2013, vol. 153 (3), 692-706
- **H. WU ; Y. ZHANG.** *Genes Dev.,* 2011, vol. 25 (23), 2436-2452
- **H. HASHIMOTO ; J.E. PAIS et al.** *Nature,* 2014, vol. 506 (7488), 391-395
- **V. VALINLUCK ; H. TSAI et al.** *Nucl Acids Res,* 2004, vol. 32 (14), 4100-4108
- **A. FREE et al.** *J Biol Chem,* 2001, vol. 276, 3353-3360
- **E. C. HULME ; M.A. TREVETHICK.** *Br J Pharmacol,* 2010, vol. 161, 1219-1237
- **S. KHRAPUNOV et al.** *Biochemistry,* 2014, vol. 53, 3379-3391
- **Y. YANG ; T. G. KUCUKKAL et al.** *ACS Chem Biol,* 2016, vol. 11, 2706-2715
- **J. CHRISTODOULOU ; A. GRIMM et al.** *Human mut,* 2003, vol. 21, 466-472